# EUROPEAN PATENT APPLICATION

(11) **EP 1 732 022 A1**
(43) Date of publication of application: **13.12.2006**
(21) Application number: 05727509.1
(22) Date of filing: 31.03.2005
(51) Int. Cl.: G06F 19/00, G06F 17/30

(54) **BASE SEQUENCE RETRIEVAL APPARATUS**

(30) Priority: 31.03.2004 JP 2004108456
(71) Applicant: Bio-Think Tank Co., Ltd., Tokyo 113-0033 (JP)
(72) Inventor: MORISHITA, Shinichi, 1790073 (JP); YAMADA, Tomoyuki, Tokyo 1130033 (JP)
(74) Representative: Betten & Resch
(86) International application number: PCT/JP2005/006397
(87) International publication number: WO 2005/096208

(57) **Abstract**

An apparatus, method, etc. that in designing of the base sequence of, for example, siRNA, realize high-speed retrieval any genes containing analogous base sequences without omission. Accordingly, retrieval is carried out in such a manner that two partial sequences of given length and any extra part arc identified from inputted base sequences, and that hamming distance being the number of corresponding bases incompatible with each other is divided and assigned to the partial sequences and extra part and out of the two partial sequences, one with an assigned number not greater is selected and retrieved.

## Description

### Field of the Invention

The present invention relates to an apparatus for searching for a gene base sequence indicating gene information, and a method thereof.

### Description of the Related Art

The study on gene information related to a base sequence was developed according to the elucidation of the DNA (Deoxyribonucleic Acid) structure by Watson and Crick. The double-helix structure of DNA is made up of a nucleotide sequence including any one of the bases of adenine (A), cytosine (C), guanine (G), or thymine (T), in which, normally, base pairs of A and T, and G and C are formed in the nucleus of a cell.

It is known that the nucleotide sequence of DNA expressing a gene (hereinafter, referred to as 'gene sequence') is transcribed to RNA (Ribonucleic Acid), and spliced, thereby generating mRNA (messenger RNA), and synthesizing protein. RNA is a nucleic acid having D-ribose as a sugar component, and adenine (A), cytosine (C), guanine (G), or uracil (U) as a base.

In recent years, the phenomenon called RNA interference was discovered. The RNA interference is a phenomenon in which the double-stranded RNA of a cell breaks mRNA having a specific sequence, thereby suppressing gene expression. This phenomenon is found in the experiment using a nematode cell at the outset. Subsequently, it was discovered that this phenomenon exists in mammal cells, and the phenomenon has been focused upon. The reason for this is that, by artificially causing RNA interference, the action of a specific gene is suppressed, so that it becomes possible to study the action of a specific gene. In addition, as a result of the discovery of RNA interference, it has become possible to develop medicine that suppresses the action of a specific gene.

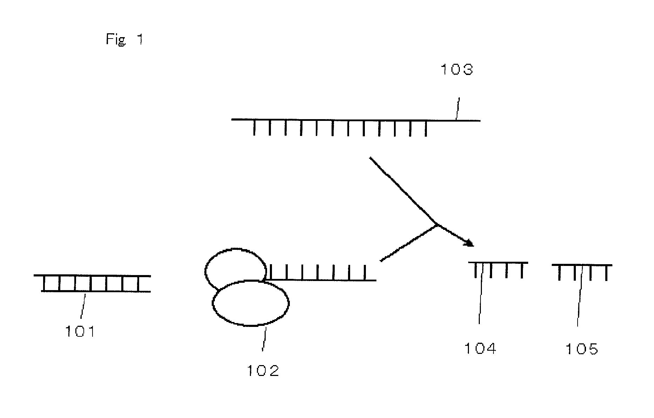

Fig. 1 is a schematic diagram showing the process of RNA interference. RNA interference occurs in the following process. ₛᵢRNA (short interfering RNA) 101, having a length of about 21 to 23 base pairs, is concatenated to multi-complex proteins, thereby forming RISC (RNA-induced silencing complex) 102. RISC is concatenated to mRNA 103, which shares homology with the ₛᵢRNA, thereby breaking the mRNA, so that the mRNA becomes dysfunctional (in Fig. 1, fragments 104 and 105 are fragments of broken mRNA). Here, the term 'two base sequences share homology' means that two base sequences are complementary, or imperfectly complementary. Here, 'complementary' means that in two entire base sequences, a pair of A and T, G and C, and A and U are perfectly formed. Accordingly, the term homology means that, in a portion of two base sequences, a pair, other than the three types of pairs A and T, G and C, and A and U, which are complementary base pairs, is formed. Note that, as described hereinbelow, it is determined whether the two base pairs share homology based on how many complementary base pairs having between two base sequences exist in what case. Therefore, in RNA interference, there are some cases, in which complementarity of more than 80%, preferably 90%, and more preferably 95%, appears, it is determined that the two base pairs share homology. Moreover, not only the percentage of complementary base pair, but also the number of series of bases appearing successively in the base sequence, is considered; the existence of homology between two base sequences is determined in some cases. Furthermore, it is known that there is a possibility of G and U forming a pair, in addition to the three types of pairs of A and T, G and C, and A and U, which are complementary base pairs, so that, considering the existence of the pair of G and U, there is a possibility of a determination of the existence of homology.

Accordingly, in order to cause RNA interference, and to suppress the action of the targeted gene, it is important to design a sequence of ₛᵢRNA. Therefore, it is important to design the sequence of ₛᵢRNA, which appears only in the targeted gene and does not share homology with the base sequence of the other genes. Accordingly, in designing the sequence of ₛᵢRNA, it is important to confirm that the target gene is the only gene having a base sequence, which is simlar to the sequence of ₛᵢRNA.

Moreover, in recent years, gene analysis or gene examination using a microarray has been carried out. The 'microarray' is a kind of DNA chip, in which oligo-DNA, having a length of 15 to 30 base pairs, is synthesized on a glass plate etc. (e.g. Non-patent document 1)

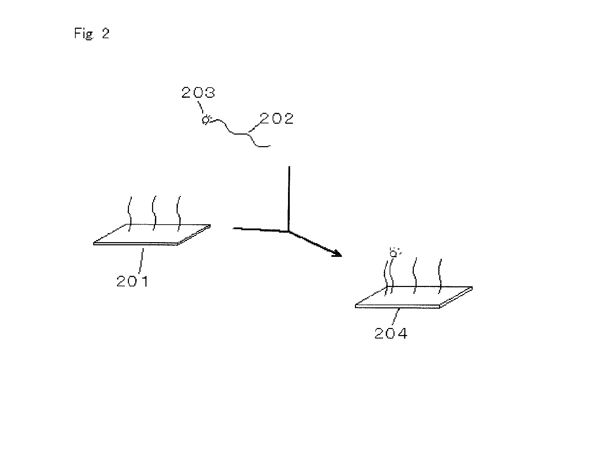

Fig. 2 is a diagram exemplifying processes of gene analysis or of gene examination etc. using microarray. When flowing DNA (202), which is fluorochrome-labeled with the label 203, on the microarray 202, in which oligo-DNA is synthesized on a glass plate etc., the oligo-DNA on the microarray sharing complementarity or homology is hybridized with the DNA (portion 204). By detecting fluorescence with the fluorescence dye of the label, it is determined at what position the DNA is hybridized with oligo-DNA, thereby determining the type of DNA (202). Although only several oligo-DNA are indicated on the microarray in Fig. 2, literally, tens of thousands of oligo-DNA exist in the 0.5 square inch area of a microarray.

Therefore, in designing a microarray, it is quite important to determine the base sequence of the oligo-DNA to be arranged on a microarray.

Conventionally, in many cases, the detection as to whether a similar base sequence exists is carried out by searching the database storing gene base sequence indicating gene information using the software called BLAST (e.g. Non-patent document 2), or algorithm called Smith-Waterman algorithm (e.g. Non-patent document 3).

Non-patent document 1: 'Genetic chemistry', Naoki Sugimoto, Kagaku-Dojin Publishing Company, Inc., 2002.

Non-patent document 2: "Basic local alignment search tool", S.F.Altschul, W.Gish, W.Miller, E.W.Myers, and D.J. Lipman, J.Mol.Biol., 215,403-410, 1990

Non-patent document 3: "Identification of common molecular subsequences", TF.Smith, and M.S.Waterman, J.Mol.Biol., 147,195-197, 1981

However, in the method using BLAST, there is a deficiency of missing the existence of a similar base sequence. In BLAST, normally, a search is carried out using a portion, in which seven of the same sequences successively exist. For this reason, in cases where the base sequence having 19 bases, for example, it is impossible to detect the base sequence having mismatches, so that the existence of a similar base sequence is missed.

Further, in Smith-Waterman algorithm, it is possible to correctly detect the existence of a similar base sequence, however a large amount of computation is required, thereby taking a long time for detection.

### SUMMARY OF THE INVENTION

It is an objective of the present invention to provide an apparatus, which requires a small amount of computation for detecting the existence of a similar base sequence, and a method thereof.

In order to achieve the above objective, in the present invention, specification of two different partial sequences, which are partial sequences of said inputted base sequence and have said predetermined length, and of the other portion is carried out; distribution and assignment of the hamming distance, which indicates the number of corresponding bases to be substituted to mismatching bases, to the partial sequence and to the other portion are carried out; selection of the partial sequence, which has a non-larger total number of substituted base sequences generated by substitution for respective said partial sequences, in which the bases of the number indicated by the assigned hamming distance are substituted to the mismatching bases, from the two partial sequences; and a search is carried out.

This makes it possible to reduce the number of base sequences generated by substitution, which is used for the search, and to reduce the amount of computation required for the search, thereby solving the above-mentioned deficiencies. In addition, an error in detecting the existence of the similar base sequence having a hamming distance, which is equal to or less than a predetermined value, is prevented, thereby solving the above-mentioned deficiencies.

According to the present invention, it becomes possible to reduce the number of base sequences generated by substitution, which is used for the search, and to reduce the amount of computation required for the search, thereby solving the above-mentioned deficiencies. In addition, the error in detecting the existence of the similar base sequence having a hamming distance, which is equal to or less than a predetermined value, is prevented, thereby solving the above-mentioned deficiencies.

### BRIEF DESCRIPTION OF THE DRAWINGS

Fig. 1 is a schematic diagram showing the process of RNA interference;
Fig. 2 is a diagram exemplifying processes of gene analysis or of gene examination etc. using microarray;
Fig. 3 is a diagram explaining the base sequence possible to be undetected by BLAST;
Fig. 4 is a functional block diagram of the apparatus for searching for base sequence of the first embodiment of the present invention;
Fig. 5 is a diagram exemplifying the base sequence of which the hamming distance is 3;
Fig. 6 is a diagram showing the definition of the hamming distance;
Fig. 7 is a diagram exemplifying the specified two partial base sequences and the other portion;
Fig. 8 is a diagram explaining the assignment of hamming distance;
Fig. 9 is a diagram explaining the assignment of hamming distance by the assignment unit, and the selection by the selection unit;
Fig. 10 is a diagram explaining the assignment of hamming distance by the assignment unit, and the selection by the selection unit;
Fig. 11 is a flow chart of the processes by the apparatus for searching for base sequence of the first embodiment of the present invention;
Fig. 12 is a functional block diagram of the apparatus for searching for base sequence of the second embodiment of the present invention;
Fig. 13 is a functional block diagram of the apparatus for searching for base sequence of the third embodiment of the present invention;
Fig. 14 is a functional block diagram of the apparatus for searching for base sequence of the fourth embodiment of the present invention;;
Fig. 15 is a functional block diagram of the apparatus for searching for base sequence of the fifth embodiment of the present invention;;
Fig. 16 is a functional block diagram of the apparatus for searching for base sequence of the sixth embodiment of the present invention;
Fig. 17 is a diagram exemplifying the program for distributing and assigning the hamming distance;
Fig. 18 is a diagram exemplifying the program for generating the substituted base sequence;
Fig. 19 is a functional block diagram of the apparatus for searching for base sequence of the tenth embodiment of the present invention;
Fig. 20 is a diagram exemplifying the table storing the repeated sequence;
Fig. 21 is a diagram exemplifying the table storing the repeated sequence information;
Fig. 22 is a flow chart of processes by the search unit of the apparatus for searching for base sequence of the tenth embodiment of the present invention;
Fig. 23 is a functional block diagram of the apparatus for searching for base sequence of the eleventh embodiment of the present invention;
Fig. 24 is a diagram showing the structure of the table, in which the inputted base sequence, the hamming distance, and the similar base sequence are correlated and stored.
Fig. 25 is a flow chart of processes 'by the determination unit and the storage unit for similar base sequence of the apparatus for searching for base sequence of the eleventh embodiment of the present invention;
Fig. 26 is a functional block diagram of the apparatus for searching for base sequence of the twelfth embodiment of the present invention;
Fig. 27 is a functional block diagram of the apparatus for generating ineffective base sequence of the thirteenth embodiment of the present invention;
Fig. 28 is a flow chart of processes by the apparatus for generating ineffective base sequence of the thirteenth embodiment of the present invention;
Fig. 29 is a schematic diagram of the processes by the apparatus of the fourteenth embodiment of the present invention;
Fig. 30 is a functional block diagram of the alignment apparatus for base sequence of the fourteenth embodiment of the present invention;
Fig. 31 is a flow chart of processes by the alignment apparatus for base sequence of the fourteenth embodiment of the present invention.

### DETAILED DESCRIPTION OF THE PREFERRED EMBODIMENTS

Embodiments of the present invention will be described hereinbelow with reference to the drawings. The present invention is not to be limited to the above embodiments and able to be embodied in various forms without departing from the scope thereof.

As the first embodiment of the present invention, an apparatus for searching for base sequence, which searches for a similar base sequence, having the same length as that of a base sequence to be inputted by using an index, which is for searching a database storing a gene base sequence indicating gene information, and is for searching for a position in said gene base sequence, at which a base sequence having a predetermined length appears, wherein specification of two different partial sequences, which are partial sequences of said inputted base sequence and have said predetermined length, and of the other portion is carried out; distribution and assignment of the hamming distance, which indicates the number of bases to be substituted to corresponding mismatching bases, to the partial sequence and to the other portion are carried out; selection of the partial sequence, which has a non-larger total number of substituted base sequences generated by substitution for respective said partial sequences, in which the bases of the number indicated by the assigned hamming distance are substituted to the mismatching bases, from the two partial sequences; and a search is carried out; will be described.

Here, the terms 'corresponding bases match' means that the corresponding two bases fulfill a predetermined binomial relation. Here, in many cases, the binomial relation means that the bases forming pairs are identical. Therefore, in terms of mathematical set theory, the binomial relation fulfills only the reflexive law. In addition, the binomial relation, by considering that G and U in the base are easily concatenated, may be used.

Note that, the 'predetermined length' is a predetermined length. This length is the length of base sequence, which is acceptable to the index for searching the database storing gene base sequence. For example, in BLAST, the predetermined length is normally 7 In addition, the 'similar base sequence' is a base sequence, which has the same length, and is similar to the base sequence to be inputted, and appears in said gene base sequence. The term 'similar' means that, for example, as descried hereinbelow, a hamming distance between the similar base sequence and the inputted base sequence becomes a provided value. Moreover, the 'said gene base sequence' is the base sequence stored by the database. Note that depending on the structure of the index, multiple predetermined lengths exist.

Such apparatus for searching for base sequence can be implemented, for example, as a server apparatus, which receives the degree of similarity with the base sequence inputted to WEB browser (e.g. hamming distance), carries out processing such as issuing query etc. to the database storing the gene base sequence, and replies a result to said WEB browser.

Therefore, it is possible to configure the respective units and the means as the components of the apparatus for searching for base sequence of the present invention by any one of hardware, software, or both hardware and software. For example, in cases where a computer is used, hardware consisting of a CPU, memory, bus, interface, peripheral devices etc., and software, operatable on the hardware, are used for implementing them.

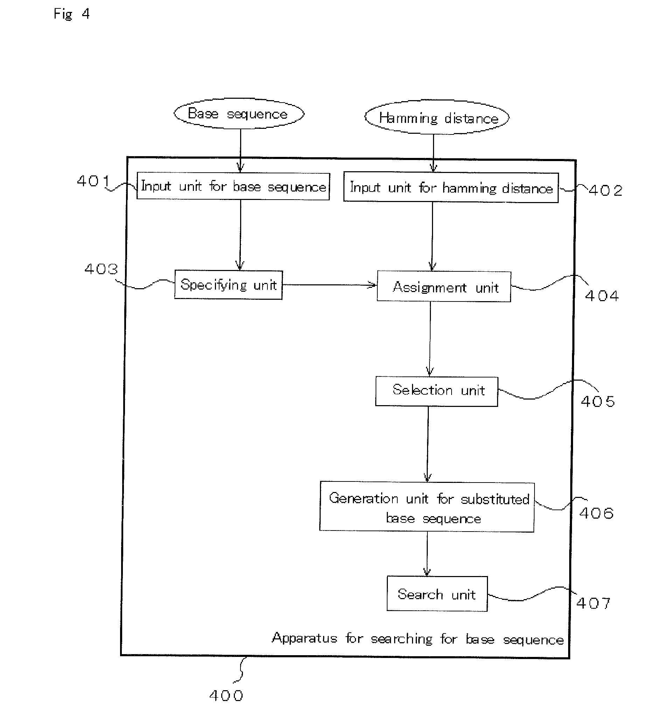

Fig. 4 is a functional block diagram of the apparatus for searching for base sequence of the first embodiment of the present invention.

The apparatus for searching for base sequence 400 comprises the input unit for base sequence 401, the input unit for hamming distance 402, the specifying unit 403, the assignment unit 404, the selection unit 405, the generation unit for substituted base sequence 406, and the search unit 407.

The 'input unit for base sequence' 401 inputs a base sequence having a length longer than the predetermined length. For example, the unit receives information indicating the base sequence inputted from the WEB browser.

The 'input unit for hamming distance' 402 inputs hamming distance for the inputted base sequence. For example, the unit receives the value inputted from the WEB browser. Here, the 'inputted base sequence' is the base sequence inputted to the input unit for base sequence 401. Further, the hamming distance is the value indicating number of bases to be substituted to mismatching bases. The hamming distance is determined for two base sequences having the same length, and is the number of mismatches between corresponding bases. By assigning the hamming distance to one base sequence, it becomes possible to determine a set of base sequences acquired by substituting the bases of the number indicated by the hamming distance to the mismatching bases.

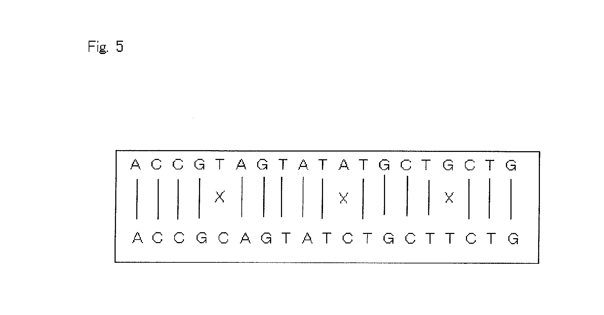

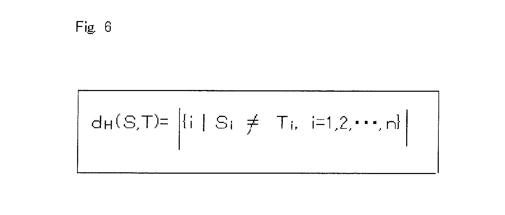

The example of the hamming distance is as follows. In Fig. 5, two base sequences consisting of 19 bases are indicated in the upper and lower portion, and 3 bases at the points indicated by 'x' do not correspond, so that the hamming distance is 3. Fig. 6 shows the definition of hamming distance. S and T are base sequences consisting of n bases, and if Sᵢ is i-th base, the hamming distance between S and T: d_{H}(S, T) is determined. Note that if U is a set, |U| indicates the number of elements, and ≠ means that bases of both sides do not match (e.g. they are not same). Therefore, the hamming distance is a non-negative integer.

The 'specifying unit' 403 specifies two different partial sequences, which are partial sequences of said inputted base sequence and have said predetermined length, and the other portion. The two partial sequences may share a common portion. Alternatively, the other portion may not exist according to conditions.

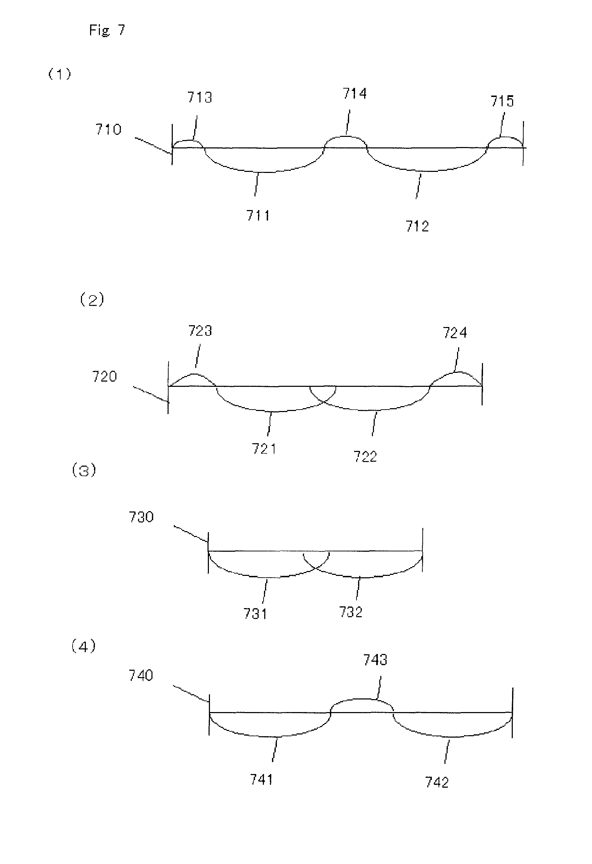

Fig. 7 is a diagram exemplifying the specified two partial base sequences and the other portion. In Fig. 7 (1), in the inputted base sequence 710, the first partial sequence 711 and the second partial sequence 712 exist so as not to share a common portion, and the other portions 713, 714, and 715 exist in the both end portions and middle of the inputted base sequence. In Fig. 7 (2), the first partial sequence 721 and the second partial sequence 724 share a common portion close to the middle, and the other portions 723, and 724 exist in the end portions of the inputted base sequence 720. In Fig. 7 (3), the first partial sequence 731 extends from the left-end of the inputted base sequence 730, the second partial sequence 732 extends from the right-end of the inputted base sequence 730, and the first partial sequence 731 and the second partial sequence 732 share a common portion close to the middle of the inputted base sequence 730. In cases where the inputted base sequence is longer than twice of the predetermined length, as shown in Fig. 7 (4), close to the middle portion of the inputted base sequence 740 corresponds to the other portion 743. Note that although the length of the first partial sequence and the length of the second partial sequence are the predetermined length, depending on the structure of the index, there is a case where multiple predetermined lengths exist as described above, and in such case, the length of the first partial sequence and the length of the second partial sequence may be the same, or may be different.

The 'assignment unit' 404 distributes and assigns the hamming distance inputted by said input unit for hamming distance to the partial sequence and to the other portion specified by said specifying unit 403. Here, the terms 'distributes and assigns the hamming distance' means that the hamming distance is distributed to non-negative integers, and the integers acquired by the distribution are assigned to the partial sequence and the other portion. Therefore, sum of the assigned values is equal to the hamming distance. Such processing can easily be implemented by a program. For example, a program, which has nested loops of the number of the partial sequence and the other portion, can implement the above processing, thereby enabling all assignments.

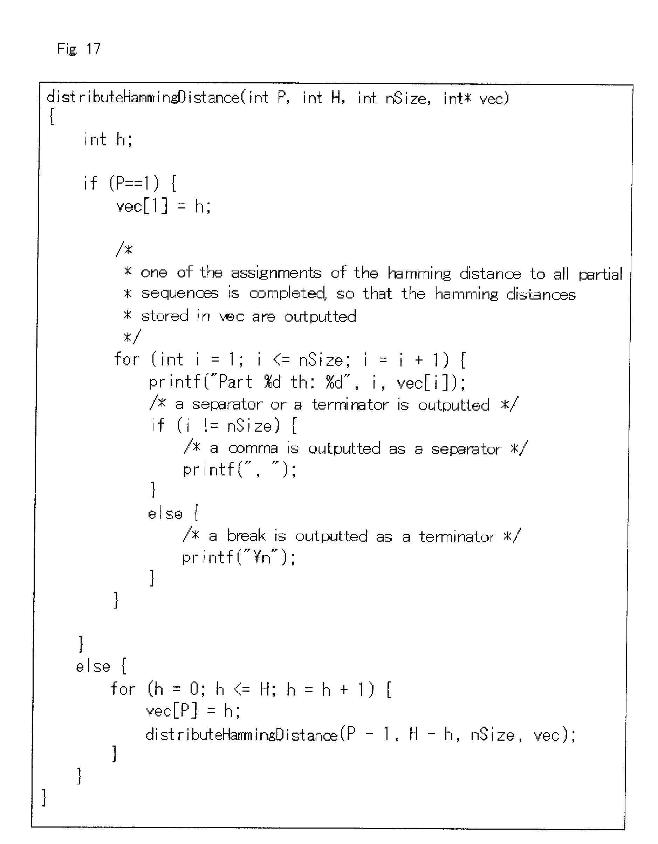

Fig. 17 is a diagram exemplifying the program for distributing and assigning the hamming distance, which is written in C. In this case, the partial sequence is specified by a number. For example, if the number of the partial sequence is P, each partial sequence is specified by P, P-1, P-2, ..., or 1, and Pth, P-1th, ..., or 1st element of predetermined sequence corresponds to the partial sequence. Then, the number of partial sequence P is set to the first parameter of 'distribute Hamming Distance', the hamming distance H to be assigned to the partial sequence of the number set to the first parameter is set to the second parameter, so that the hamming distance is assigned to the partial sequence respectively specified by P, P-1, P-2, ..., 1. Note that the total number of partial sequences is set to the third parameter of 'distributeHammingDistance', and the predetermined sequence is set to the fourth parameter of 'distributeHammingDistance'. The detailed operation of 'DistributeHammingDistance' is as follows. Therefore, if the predetermined sequence is indicated by vec, with respect to each one call of 'distributeHammingDistance', the hamming distance assigned to the partial sequence is substituted to any one of vec[P], vec[P-1], vec[P-2], ..., vec[1], and a recursive call of 'distributeHammingDistance' is carried out. For example, in cases where, in one of the calls of 'distributeHammingDistance', the hamming distance assigned to the partial sequence q is substituted to vec[q], if q is not 1, q-1 is set to the first parameter of 'distributeHammingDistance', thereby carrying out a recursive call of 'distributeHammingDistance'. Finally, if the hamming distance is substituted to vec[1], one of the assignments of the hamming distance to all partial sequences is completed, so that the values of vec[P], vec[P-1], vec[P-2], ..., vec[1] are outputted using the function 'printf'. Of course, it is easy for a person skilled in the art to implement selection of partial sequence as described below, in which the assignment of the hamming distance is stored to data structure configured in a memory, and the selection unit 405 refers to the data structure, instead of output using 'printf'.

Note that in the program of Fig. 17, 'int' indicates integer data type, and for example, 'int h;' means that the parameter h has a value of integer data type. Further, 'for (S1; S2; S3){S4}' means that S1 is executed initially, and as long as the condition of S2 is fulfilled, the execution of S3 subsequent to execution of S4 is repeated.

Note that, although it has been exemplified in Fig. 17, 'DistributeHammingDistance' is defined as follows: 'distributeHammingDistance(int P, int H, int nSize, int* vec) { int h; if (P==1) {vec[1] = h; /*one of the assignments of the hamming distance to all partial sequences is completed, so that the hamming distances stored in 'vee' arc outputted*/ for (int i = 1; i <= nSize; i = i + 1) { printf("Part %d th: %d", i, vec[i]); /*a separator or a terminator is outputted*/ if (i != nsize) { /*a comma is outputted as a separator*/ printf(", "); } else {/*a break is outputted as a terminator*/ printf("¥n"); } else {for (h = 0; h <= H; h = h + 1) { vec[P] = h; distributeHammingDistance(P - 1, H - h, nSize, vec); } } }'. Thus, by executing the recursive call, the loops of the number of the partial sequences and the other portions are nested.

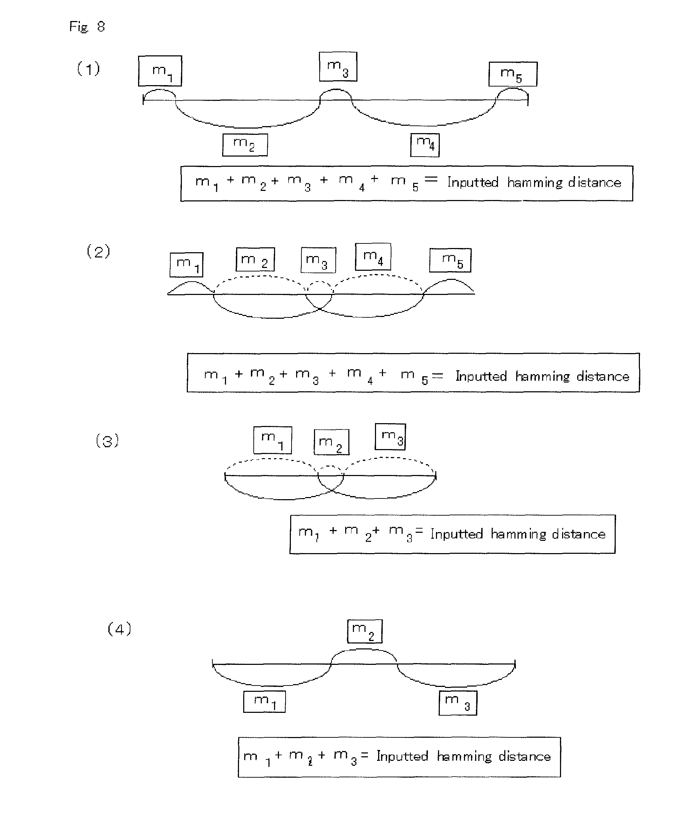

Fig. 8 is a diagram explaining the assignment of hamming distance, corresponding to the cases of Fig. 7 (1) to (4). In Fig. 8 (1), if the value m₁, m₂, m₃, m₄, and m₅ are assigned to the portions from the left-end of the inputted base sequence (i.e., assigned to the other portion, to the first partial sequence, to the other portion, to the second sequence, and to the other portion), the sum of the m₁, m₂, m₃, m₄, and m₅ is the inputted hamming distance. Here, the 'inputted hamming distance' is the hamming distance inputted by the input unit for hamming distance 402.

Similarly, in Fig. 8 (2), if the m₁, m₂, m₃, m₄, m₅ are assigned from the left-end of the inputted base sequence (i.e., the other portion, the left portion of the first partial sequence, the common portion, the right portion of the second sequence, and the other portion), the sum of the m₁, m₂, m₃, m₄, m₅ is the inputted hamming distance.

In Fig. 8 (3), if the m₁, m₂, m₃ are assigned from the left-end of the inputted base sequence (i.e., the left portion of the first partial sequence, the common portion, and the right portion of the second sequence), the sum of them is the inputted hamming distance.

In Fig. 8 (4), if the m₁, m₂, m₃ are assigned from the left-end of the inputted base sequence (i.e., the first partial sequence, the other portion, and the second sequence), the sum of them is the inputted hamming distance.

The 'selection unit' 405 selects the partial sequence having non-larger total number of substituted base sequences generated by substitution for said partial sequence, in which the bases of the number indicated by the hamming distance assigned by said assignment unit are substituted to the mismatching bases, from the two partial sequences specified by said specifying unit 403. This total number can be computed by the formula: (number of mismatching bases)^{(hamming distance)} × (predetermined _{length)}C_{(hamming} distance), the selection is carried out based on a result of this computation. However, in many cases, the partial sequence having non-larger hamming distance assigned by the assignment unit 404. Therefore, for example, in the case of Fig. 8 (1), m₂ and m₄ are compared, and if m₂ is non-larger, the first partial sequence is selected. Meanwhile, if m₄ is smaller, the second partial sequence is selected. However, for example, if to two partial sequences having predetermined length 4, the hamming distance 3 is assigned to the one and 4 is assigned to the other, the total number of substituted base sequences of the one partial sequence is computed as follows. Therefore, if a mismatching base is a different base, the number of base types is 4, so that the number of different types from one base is (4-1), and the total number of substituted base sequences of the one partial sequence is (4-1)³₄C₃ = 108. However, the total number of substituted base sequences of the other partial sequence is (4-1)⁴₄C₄ = 81, so that there exists the case where the number of the other substituted base sequences, to which a larger hamming distance has been assigned, is smaller. Therefore, the smallness or largeness of the total number of substituted base sequences does not correspond to the smallness or largeness of the hamming distance, thereby needing attention. Note that, in order to simplify the explanation, assuming that the total number of the substituted base sequences, to which a non-larger hamming distance has been assigned by the assignment unit 404, is non-larger, the explanation will be described, hereinbelow.

Similarly, in Fig. 8 (2), m₂+m₃, and m₃+m₄ are compared, and, for example, if m₂+m₃ is non-larger, the first partial sequence is selected. On the contrary, if m₃+m₄ is smaller, the second partial sequence is selected.

In addition, in Fig. 8 (3), m₁+m₂, and m₂+m₃ are compared, and, for example, if m₁+m₂ is non-larger, the first partial sequence is selected. Conversely, if m₂+m₃ is smaller, the second partial sequence is selected.

In addition, in Fig. 8 (4), m₁ and m₃ are compared, and, for example, if m₁ is non-larger, the first partial sequence is selected. On the other hand, if m₃ is smaller, the second partial sequence is selected.

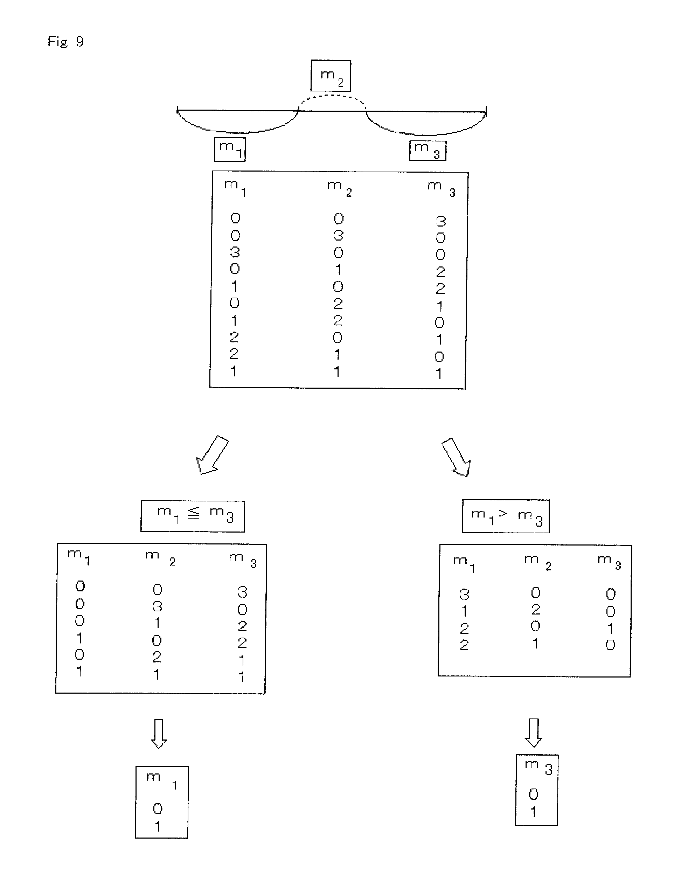

Fig. 9 is a diagram explaining the assignment of hamming distance by the assignment unit, and the selection by the selection unit, in the case where the inputted hamming distance is 3, and the partial sequence and the other portion are specified as in Fig. 7 (4). In Fig. 9, in order to simplify the explanation, the case where the sum of m₁+m₂+m₃ is equal to the inputted hamming distance 3 is exemplified. Although there are 10 combinations, in which the sum of m₁+m₂+m₃ is 3, if the selection unit 405 carries out, for example, selecting the cases of m₁ ≦ m₃, 6 combinations are acquired as a result. Further, the duplications as to the value of m₁ are eliminated, so that 0 and 1 are acquired. The same is applied to the second partial sequence and m₁. Consequently, m₃ has 0 and 1. Note that the cases of m₁=m₃ are excluded, so that the number of cases of selecting m₁>m₃ is less than the number of selecting the case of m₁ ≦ m₃. This means that, by the operations of the generation unit for substituted base sequence 406 and of the search unit 407, which will be described hereinbelow, as to the case where m₁ is 0 or 1, and also as to the case where m₃ is 0 or 1, the generation unit for substituted base sequence 406 generates the substituted base sequence and the search unit carries out search referring to the index, so that the searches as to the 10 cases, in which the sum of m₁+m₂+m₃ is 3, are covered.

In addition, the hamming distance inputted to the input unit for hamming distance is assigned to multiple portions, and the non-larger case such as m₁≦m₃ or m₁>m₃ is selected, so that the combination of values of m₁, and m₃ acquired as described above can be acquired if it is less than the hamming distance inputted to the input unit for hamming distance. Therefore, if selection as to the case of hamming distance H is carried out, selection as to the case where the hamming distance less than H is inputted to the input unit for hamming distance is also carried out.

Therefore, similarly, the case where the sum of m₁+m₂+m₃ is less than 3 can be processed. Thus, according to the present invention, it becomes possible to process not only the case where the sum of m₁+m₂+m₃ is the value provided by the hamming distance, but also the case where it is less than the value, at once.

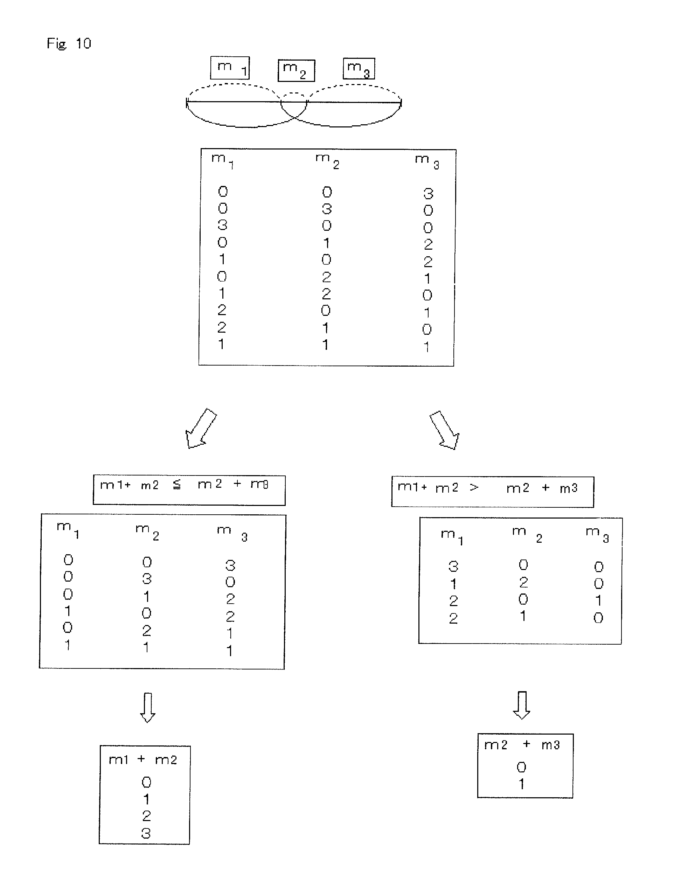

Fig. 10 is a diagram explaining the assignment of hamming distance by the assignment unit, and the selection by the selection unit, in the case where the inputted hamming distance is similarly 3, and the partial sequence and the other portion are specified as in Fig. 7 (3). Similarly, in Fig. 10, in order to simplify the explanation, the case where the sum of m₁+m₂+m₃ is equal to the inputted hamming distance 3 is exemplified. Although there are similarly 10 combinations, in which the sum of m₁+m₂+m₃ is 3, if the selection unit 405 carries out, for example, selecting the cases of m₁+m₂≦m₂+m₃, 6 combinations are acquired as a result. Further, the duplications as to the value of m₁+m₂ are eliminated, so that the 4 combinations as to 0, 1, 2, and 3 are acquired. The same is applied to the second partial sequence and m₂+m₃. However, the cases of m₁+m₂≦m₂+m₃ are excluded, so that the number of the cases of selecting m₁+m₂>m₂+m₃ is less than the number of selecting the cases of m₁+m₂≦m₂+m₃. Consequently, m₂+m₃ has 0 and 1. This means that, by the operations of the generation unit for substituted base sequence 406 and of the search unit 407, which will be described hereinbelow, as to the cases where m₁+m₂ is 0 or 1, and also as to the cases where m₂+m₃ is 0 or 1, the generation unit for substituted base sequence 406 generates the substituted base sequence and the search unit carries out search referring the index, so that the searches as to the 10 cases, in which the sum of m₁+m₂+m₃ is 3, are covered.

In addition, the hamming distance inputted to the input unit for hamming distance is assigned to multiple portions, and the non-larger case such as m₁+m₂>m₂+m₃, or m₁+m₂≦m₂+m₃ is selected, so that the combination of values of m₁+m₂, and m₂+m₃ acquired, as described above, can be acquired if it is less than the hamming distance inputted to the input unit for hamming distance. Therefore, if selection as to the case of hamming distance H is carried out, selection as to the case where the hamming distance less than H is inputted to the input unit for hamming distance is also carried out.

The 'generation unit for substituted base sequence' 406 generates a substituted base sequence, which has the hamming distance assigned by said assignment unit, for the partial sequence selected by said selector. Therefore, the bases of the number indicated by the hamming distance assigned by the assignment unit 404 among the partial sequences selected by the selection unit 405 are substituted to the mismatching bases, thereby generating the substituted base sequences. For example, in the case of Fig. 9, as to the first partial sequence, the partial sequences having the hamming distance 0 or 1 are generated as the substituted base sequences. In addition, as to the second partial sequence, the partial sequences having the hamming distance 0 or 1 arc generated as the substituted base sequences. If the hamming distance is 0, it corresponds to the first partial sequence itself, and if the hamming distance is 1, any one of the bases in the first partial sequence is substituted to the mismatching base, thereby generating the substituted base sequence. Similarly, in the case of Fig. 10, as to the first partial sequence, the partial sequences having the hamming distance 0, 1, 2, or 3 are generated as the substituted base sequences. In addition, as to the second partial sequence, the partial sequences having the hamming distance 0, or 1 are generated as the substituted base sequences. In this case, since the inputted hamming distance is 3, it seems inefficient to generate the substituted base sequence having the hamming distance 3. However, since 3 has been assigned to m₂, it is only necessary to generate the substituted base sequence having the hamming distance 3 for the common portion of the first and second partial sequence. If the length of that portion is short, the total number of the substituted base sequence having the hamming distance 3 is limited. Thus, in the case where the first and second partial sequence share the common portion, by considering the hamming distance assigned to the common portion and to the non-common portion, and generating the substituted base sequence for the common portion and to the non-common portion, respectively, it becomes possible to improve efficiency of generating the substituted base sequence.

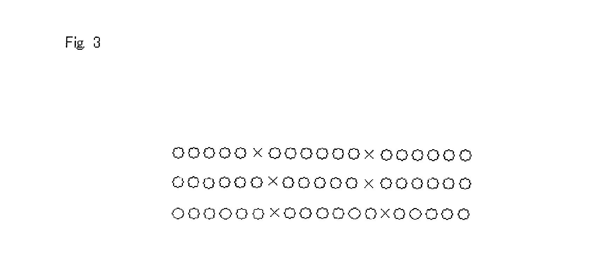

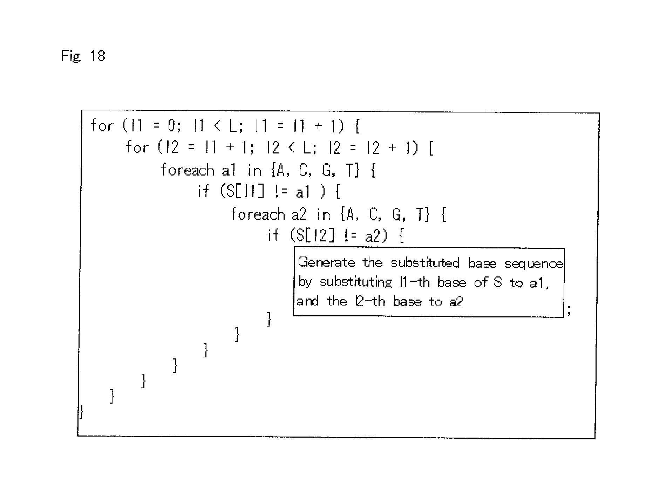

The program for generating the substituted base sequence is easily created. For example, a program having nested loop is generated, the position of the partial sequence having a base to be substituted to a mismatching base is specified by the outside loop, and the base of the position specified by the outside loop is substituted to a mismatching base by the inside loop. If the predetermined length is L, and difference between the bases is defined to mismatch, in the case of Fig. 9, 1+3_{L}C₁ substituted base sequences are generated. Although in the case of Fig. 10, 1+3_{L}C₁+3²_{L}C₂+3³_{L}C₃ substituted base sequences are generated, since L is generally smaller than the value of the length of the inputted base sequence, the computational amount required for this generation is smaller than the computational amount for all base sequences having the hamming distance 3 for the inputted base sequence. Fig. 18 is a diagram exemplifying the program for generating the substituted base sequence of the partial sequence in cases where the hamming distance 2 is assigned to the partial sequence having the length L for the sequence S. In this program, the suffix is 0, any one of the symbols A, C, G, or T, indicating a base, is stored in S[0], S[1], ..., S[L-1]. Further, for example, 'foreach a1 in {A, C, G, T} {S}' indicates that S is executed by continuously changing the value of the parameter a1 to A, C, G, and T. In Fig. 18, 'for(11=0;11<L;11=11+1)' and 'for(12=0;12<L;12=12+1)' corresponds to the above 'outside loop', and 'foreach a1 in {A, C, G, T}' and 'foreach a2 in {A, C, G, T}' corresponds the above 'inside loop'. Although exemplified in Fig. 17, the program is defined as follows: for (11 = 0; 11 < L; 11 = 11 + 1) { for (12 = 11 + 1; 12 < L; 12 = 12 + 1) { foreach a1 in {A, C, G, T} { if (S[11] != a1) { foreach a2 in {A, C, G, T} { if (S[12] != a2) { generate the substituted base sequence by substituting 11-th base of S to a1, and the 12-th base to a2; }}}}}}.

The 'search unit' 407 carries out search using said index and the substituted base sequence generated by said generator for substituted base sequence as a search key. In many cases, the index is implemented using the hush method. The 'said index' is an index for searching for an appearance of the base sequence having the predetermined length in the database storing the gene sequence. By the search using such index, generally, the position information of appearance of the substituted base sequence (e.g., information indicating that the base at the end of substituted base sequence corresponds to the base of what number from 5'-end of DNA).

If the apparatus for searching for base sequence comprises a database storing a gene base sequence, the search unit 407 sends a query to the database. Further, if there is another server comprising such a database, the search unit 407 may send a query to the database, and may receive a result.

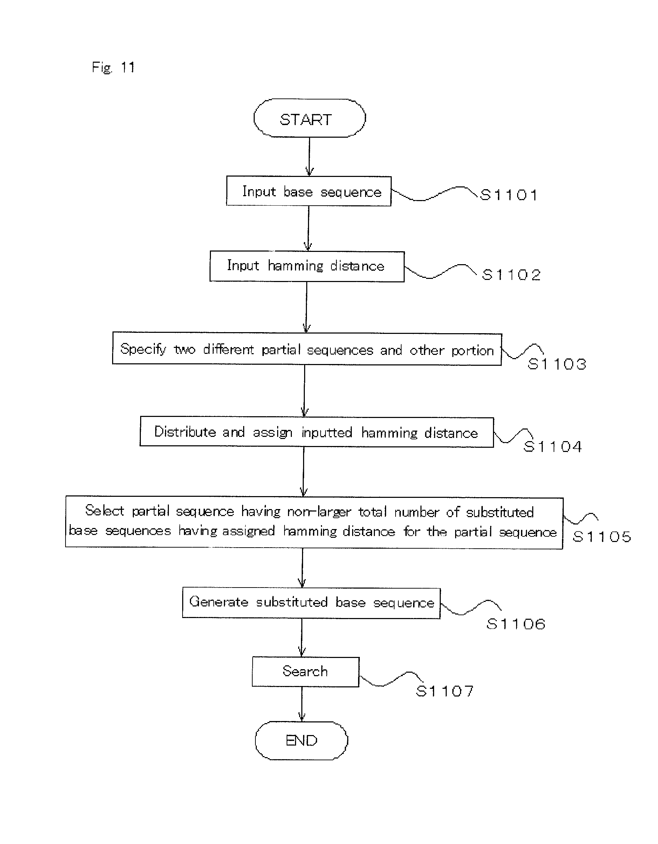

Fig. 11 is a flow chart of the apparatus for searching for base sequence in Fig. 4 of the first embodiment. In step S1101, the input unit for base sequence 401 etc., inputs a base sequence (the step of inputting base sequence). In the step S1102, the input unit for hamming distance 402 etc. inputs hamming distance (the step of inputting hamming distance). In the step S1103, the specifying unit 403 etc. specifies two different partial sequences and the other portion (the step of specifying). In the step S1104, the assignment unit 404 etc. distributes and assigns the inputted hamming distance (the step of assigning). In the step S1105, the selection unit 405 etc. selects the partial sequence having non-larger total number of substituted base sequences having the hamming distance assigned by the step of assigning without generating the overlapping portion (the step of selecting). In the step S 1106, the generation unit for substituted base sequence 406 etc. generates a substituted base sequence (the step of generating substituted base sequence). In the step S1107, the search unit 407 etc. carries out search (the step of searching).

Therefore, the apparatus for searching for base sequence can be regarded as the apparatus for using the method for searching for base sequences comprising, the step of inputting base sequence, the step of inputting hamming distance, the step of specifying, the step of assigning, the step of selecting, the step of generating substituted base sequence, and the step of searching.

Note that the flow chart in Fig. 11 is an example. As to one of the base sequences inputted by the step S1101, by changing the hamming distance to be inputted by the step S1102 as 0, 1, 2, 3, 4 and so on, the other step may be repeatedly carried out. In addition, after carrying out the step S1101, the step S1103 may be carried out, the step S1102 may be carried out, and the other steps may be carried out. In addition, after carrying out the step S1101 to 1104 changing the hamming distance to be inputted as 0, 1, 2, 3, 4, the steps after S1105 may be carried out. This makes efficient computation without repeating search using the same partial sequence.

According to the first embodiment, it becomes possible to reduce the computational amount required for search, and to carry out search for similar base sequence having the hamming distance, which is equal to a predetermined value, less than the predetermined value, or any combination of values, without failure.

Note that, in the present specification, the configurations indicated in the functional block diagram of Fig. 4 can be implemented as hardware by a CPU, memory, other LSI of any computer etc. Moreover, they are implemented as software by a program loaded to a memory etc. Furthermore, they may be implemented by a combination of hardware and software. Specifically, in cases where they are implemented by software, the program configuring the program is recorded to various recording mediums and may be automatically read by a computer to implement the apparatus for searching for base sequence according to necessity. Here, the 'recording medium' may include any 'transportable type physical medium' such as a flexible disk, an optical disk, a ROM, a EPROM, a EEPROM, a CD-ROM, a MO, a DVD, a flash disk, any 'fixed type physical medium' such as ROM, RAM, or HD mounted in various computer systems, or 'communication medium' for storing the program for a short period such as a communication line or carrier wave in the case of transmitting the program via network typified by LAN, WAN, or Internet. Note that the above computer is not limited to a mainframe computer, and may be an information processing device such as a workstation, or a personal computer. Further, to such an information processing device, peripheral devices such as a printer or a scanner may be connected.

In addition, the 'program' means a data processing method described by any language or description method, and any format such as source code or binary code etc. may be allowed. Note that the 'program' is not necessarily limited to a program having a single configuration, and may include a program having a distributed configuration as multiple modules or library, and a program, which cooperates with other programs typified by operating system, and implements function. Note that, in the apparatus for searching for base sequence, general configuration or process may be used for the specific configuration for reading the recording medium, the reading means, or install process after reading etc.

For example, the input unit for base sequence 401, the input unit for hamming distance 402, the specifying unit 403, the assignment unit 404, the selection unit 405, the generation unit for substituted base sequence 406, and the search unit 407 of the apparatus for searching for base sequence of the first embodiment can be implemented as modules configuring the program, respectively. Such modules are definitely controlled by a CPU of the computer.

Although not indicated in the drawing, the apparatus for searching for base sequence may be communicably connected to the external system for providing the external program for searching the external database of information of the base sequence of gene etc. via the communication network such as the internet. By this configuration, a website for carrying out the external program is provided. The external system may be configured as a WEB server or ASP server etc. For example, the apparatus for searching for base sequence may be communicably connected to the external system. Although the configuration of the communication network is not specifically limited, for example, it is configured by a communication device such as a router, and wired or wireless communication line such as an exclusive line.

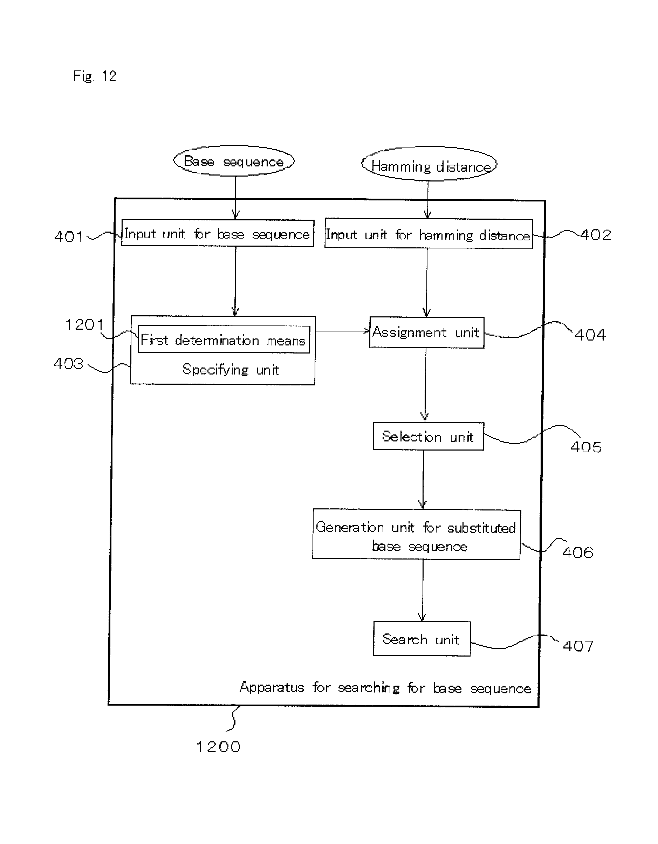

Fig. 12 is a functional block diagram of the apparatus for searching for base sequence of the second embodiment of the present invention.

The apparatus for searching for base sequence 1200 comprises the input unit for base sequence 401, the input unit for hamming distance 402, the specifying unit 403, the assignment unit 404, the selection unit 405, the generation unit for substituted base sequence 406, and the search unit 407. The specifying unit 403 comprises the first specifying means 1201. Therefore, the apparatus for searching for base sequence of the second embodiment has the configuration in which the specifying unit of the apparatus for searching for base sequence of the first embodiment comprises the first specifying means.

In the 'first specifying means' 1201, if number of bases of the base sequence inputted by said input unit for base sequence is equal to or less than twice of said predetermined length, one end of one partial sequence of said two partial sequences is conformed to the other end of said inputted base sequence, so that the other portion does not exist and is not specified. The other portion does not exist and is not specified, so that the assignment unit docs not assign the hamming distance to the other portion.

Therefore, the first specifying means specifies the first partial sequence and second partial sequence as shown in Fig. 7 (3). Accordingly, the above case has already been described in the first embodiment, so that the description will be omitted.

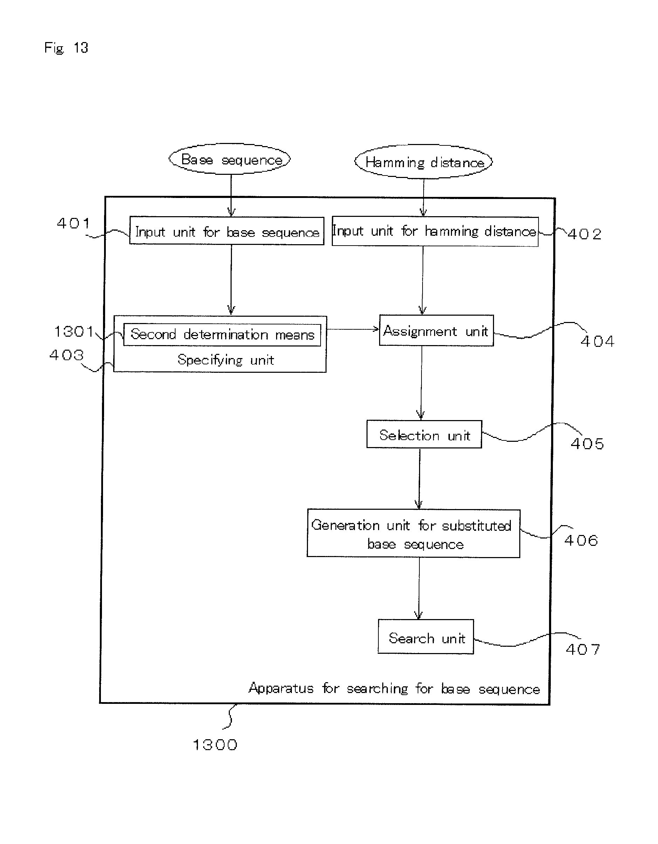

Fig. 13 is a functional block diagram of the apparatus for searching for base sequence of the third embodiment of the present invention.

The apparatus for searching for base sequence 1300 comprises the input unit for base sequence 401, the input unit for hamming distance 402, the specifying unit 403, the assignment unit 404, the selection unit 405, the generation unit for substituted base sequence 406, and the search unit 407. The specifying unit 403 comprises the second specifying means 1301. In addition, the specifying unit 403 may comprise the first specifying means described in the second embodiment. Therefore, the apparatus for searching for base sequence of the third embodiment has the configuration in which the specifying unit of the apparatus for searching for base sequence of the first or second embodiment comprises the second specifying means.

In the 'second specifying means' 1301, if number of bases of the base sequence inputted by said input unit for base sequence is more than twice of said predetermined length, said two partial sequences do not overlap each other, and said two partial sequences are specified. In this case, the other portion may be one, or two. For example, two partial sequences are specified so as to be arranged at the right and left ends of the inputted base sequence, or the inputted base sequence is specified, so that two partial sequences are concatenated.

Therefore, the second specifying means specifies the first partial sequence and second partial sequence as shown in Fig. 7 (4). Accordingly, the above case has already been described in the first embodiment, so that the description will be omitted.

As the fourth embodiment of the present invention, the apparatus for searching for base sequence, which acquires the similar base sequence candidate and determines the hamming distance between the similar base sequence candidate and the inputted base sequence based on the search result by the search unit.

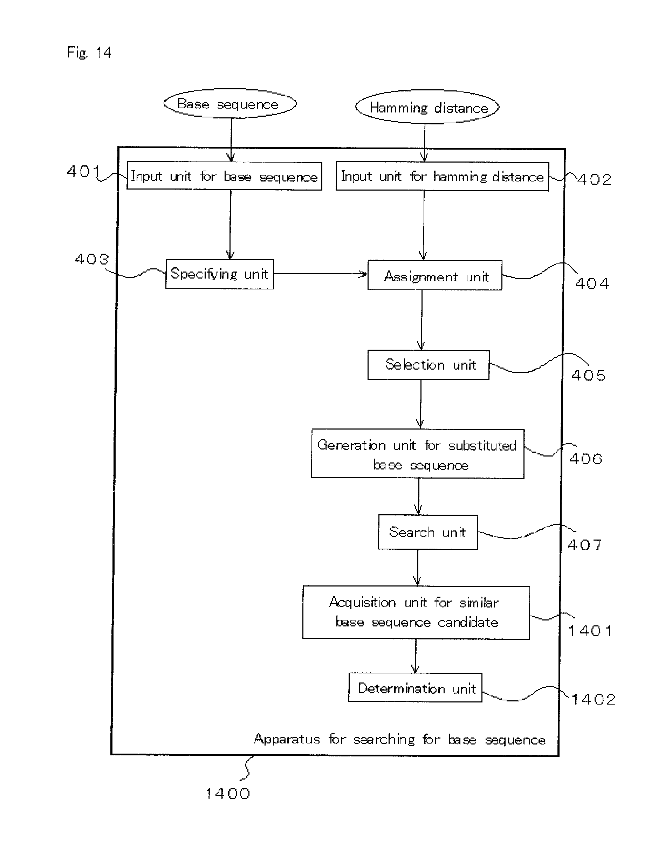

Fig. 14 is a functional block diagram of the apparatus for searching for base sequence of the fourth embodiment of the present invention.

The apparatus for searching for base sequence 1400 comprises the input unit for base sequence 401, the input unit for hamming distance 402, the specifying unit 403, the assignment unit 404, the selection unit 405, the generation unit for substituted base sequence 406, and the search unit 407, the acquisition unit for similar base sequence candidate 1401, and the determination unit 1402. In addition, the specifying unit 403 may comprise one or both of the first specifying means described in the second embodiment or the second specifying means described in the third embodiment. Therefore, the apparatus for searching for base sequence of the fourth embodiment has the configuration in which the apparatus for searching for base sequence of any one of the first to third embodiments comprises the acquisition unit for similar base sequence candidate 1401, and the determination unit 1402

The 'acquisition unit for similar base sequence candidate' 1401 acquires a similar base sequence candidate based on the search result by said search unit 407. The 'similar base sequence candidate' is a base sequence including said substituted base sequence and appearing in a gene base sequence. Concretely speaking, for example, if the search is carried out by the substituted base sequence of the first partial sequence, and the position of the base at the end of the substituted base sequence is detected, the gene base sequence having the same length as that of the inputted base sequence is acquired by considering the positional relation of the first partial sequence in the inputted base sequence. Therefore, if the position of the left-end base of the first partial sequence is acquired by the search, the gene base sequence having the same length as that of the inputted base sequence is acquired, in which the length is measured from the left-end position of the other portion adjacent to the left side of the first partial sequence (if the other portion does not exist, the value is 0). Similarly, in cases where the search for the substituted base sequence of the second partial sequence is carried out, the gene base sequence having the same length as that of the inputted base sequence is acquired, in which the length is measured from the right-end position of the other portion adjacent to the right side of the second partial sequence. This acquisition is carried out by searching the database. If the apparatus for searching for base sequence comprises such a database, the similar base sequence candidate is acquired from the database, and if the other server comprises such a database, the query is transmitted to the server, and the similar base sequence candidate is acquired.

The 'determination unit' 1402 determines whether the hamming distance between the similar base sequence candidate acquired by said acquisition unit for similar base sequence candidate and said inputted base sequence is less than or equal to the hamming distance inputted by said input unit for hamming distance. This determination can be carried out by comparing the inputted base sequence with similar base sequence candidate from the end base.

In the processing flow of the apparatus for searching for base sequence of the fourth embodiment, after the step S1107 indicated in Fig 11, the step of acquiring similar base sequence candidate, and the step for determining whether the hamming distance between the similar base sequence candidate and the inputted base sequence is equal to the inputted hamming distance are carried out.

According to the fourth embodiment, it becomes possible to acquire the base sequence similar to the inputted base sequence, so that, for example, it becomes possible to acquire information of a gene, which can be inactivated, other than the target gene, which is inactivated by ₛᵢRNA.

In the fifth embodiment of the present invention, the apparatus for searching for base sequence, which is able to specify a combination of mismatching bases.

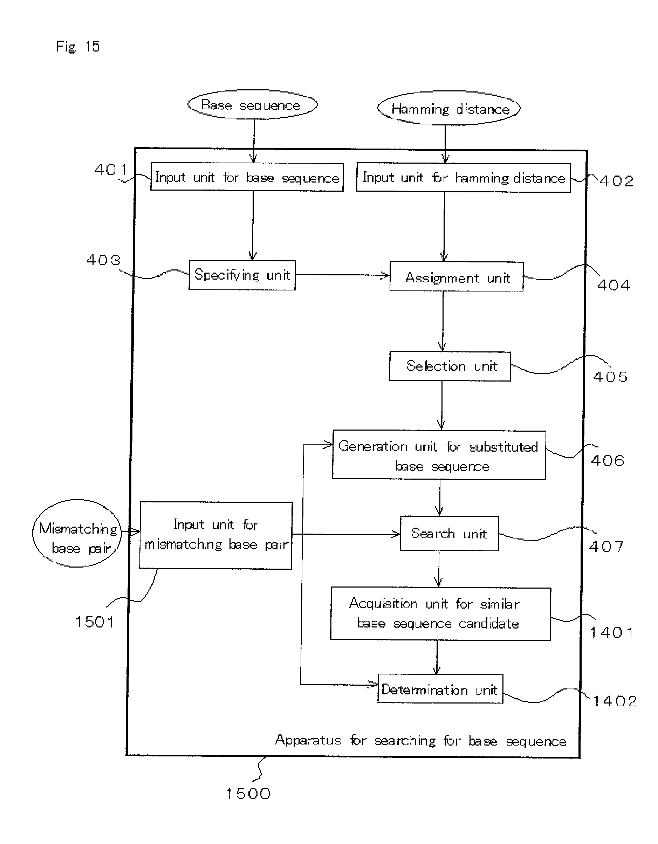

Fig. 15 is a functional block diagram of the apparatus for searching for base sequence of the fifth embodiment of the present invention.

The apparatus for searching for base sequence 1500 comprises the input unit for base sequence 401, the input unit for hamming distance 402, the specifying unit 403, the assignment unit 404, the selection unit 405, the generation unit for substituted base sequence 406, and the search unit 407, the acquisition unit for similar base sequence candidate 1401, the determination unit 1402, and the input unit for mismatching base pair 1501. In addition, the specifying unit 403 may comprise one or both of the first specifying means described in the second embodiment or the second specifying means described in the third embodiment. Therefore, the apparatus for searching for base sequence of the fifth embodiment has the configuration in which the apparatus for searching for base sequence of fourth embodiment comprises the input unit for mismatching base pair 1501.

The 'input unit for mismatching base pair' 1501 specifies mismatching base pair. For example, the text information indicating base pair to be determined to be mismatching is inputted. Alternatively, the base pair to be determined to be mismatching may be indirectly specified by inputting the base pair to be determined to be matching (e.g., G and U).

In the fifth embodiment, the search is carried out by the search unit based on the base pair inputted to the input unit for mismatching base pair 1501, and the hamming distance is acquired. For example, based on the base pair inputted to the input unit for mismatching base pair 1501, the substituted base sequence is generated by the generation unit for substituted base sequence 406, and the database for search is selected by the search unit 407, and the hamming distance is acquired by the determination unit 1402.

According to the fifth embodiment, it becomes possible to consider the base pair such as G and U., which are possible to be weakly concatenated, and to accurately design the base sequence.

In the sixth embodiment of the present invention, the apparatus for searching for base sequence, which is able to specify distribution of matching of bases between the inputted base sequence and the similar base sequence.

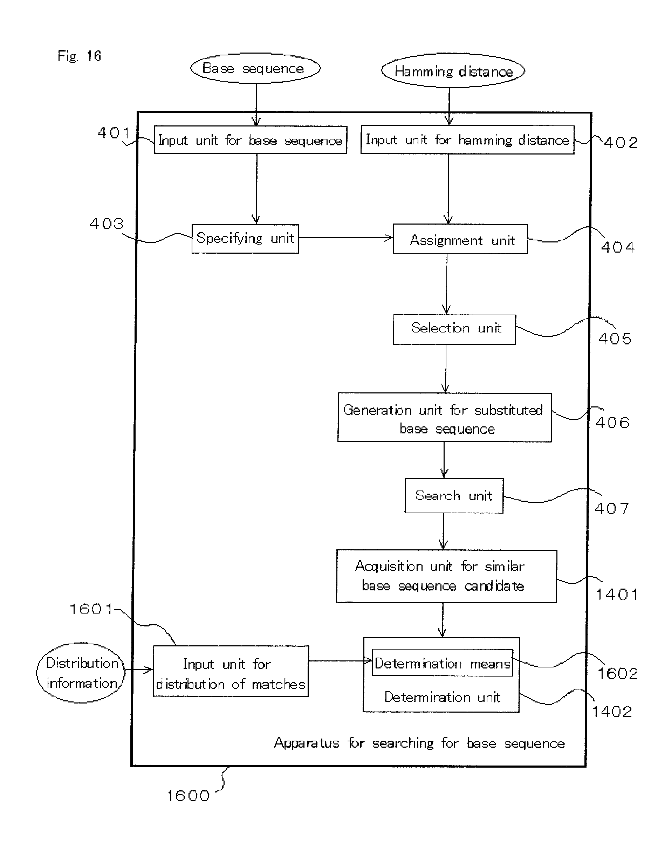

Fig. 16 is a functional block diagram of the apparatus for searching for base sequence of the sixth embodiment of the present invention.

The apparatus for searching for base sequence 1600 comprises the input unit for base sequence 401, the input unit for hamming distance 402, the specifying unit 403, the assignment unit 404, the selection unit 405, the generation unit for substituted base sequence 406, and the search unit 407, the acquisition unit for similar base sequence candidate 1401, the determination unit 1402, and the input unit for distribution of matching 1601. Further, the determination unit 1402 comprises the determination means 1602. In addition, the apparatus for searching for base sequence 1600 may comprise the input unit for mismatching base pair described in the fifth embodiment. Therefore, the apparatus for searching for base sequence of the sixth embodiment has the configuration in which the apparatus for searching for base sequence of fourth or fifth embodiment comprises the input unit for distribution of matching 1601, and the determination unit 1402 comprises the determination means 1602.

The 'input unit for distribution of matching' 1601 inputs distribution information indicating distribution of matches between the corresponding bases in the base sequence inputted by said input unit for base sequence 401 and in the similar base sequence. An example of the distribution information includes information indicating that the occurrence of mismatches of bases in the 5'-end side is less, many, or mismatches of bases occur at almost regular intervals. The distribution information, for example, may be a program for determining the distribution of matches of bases, or information for selecting types of distribution of matches of bases, which is preliminarily determined.

The 'determination means for distribution' 1602 determines whether the distribution information inputted by said input unit for distribution of matches 1602 is fulfilled.

The 'determination unit' 1402 may indicate, for example, a determination result by the determination means for distribution with the similar base sequence.

According to the sixth embodiment, it becomes possible to accurately design the base sequence.

In the apparatus for searching for base sequence of the seventh embodiment, the distribution information inputted by said input unit for distribution of matches 1601 of the apparatus for searching for base sequence of the sixth embodiment is the lower limit of length of successive matching bases between the corresponding bases in the base sequence and in the similar base sequence.

Between two base sequences, there is the case where if corresponding bases successively match, hybridization occurs, even if the mismatching base exists in the corresponding bases. According to the seventh embodiment, it becomes possible to detect the similar base sequence, which is possible to be hybridized, by specify the lower limit of length of successive matching bases.

The apparatus for searching for base sequence of the eighth embodiment is according to any one of the first to seventh embodiments, wherein
the length of the base sequence inputted by said input unit for base sequence is 15 to 60, preferably, 15 to 25, and said predetermined length is 11 to 14.

The length of the base sequence inputted by said input unit for base sequence is 15 to 60, preferably, 15 to 25, and said predetermined length is I 1 to 14, so that it becomes possible to accurately design the base sequence by the apparatus for searching for base sequence of the eighth embodiment. Further, in the database, which the inventor used for a benchmark test, when the length of the inputted base sequence is 19 or 20, in cases where the predetermined length is 11 to 14, the search was carried out at the highest speed. The reason for this is that if the predetermined length is small, the number of the similar base sequences becomes large, and if the predetermined length is large, the computational amount required for generation of the substituted base sequence by the generation unit for substituted base sequence becomes large, and mistakenly hits upon sending query to the hash table configuring the index increases. Therefore, the query for a base, which docs not originally exist in the database, increases, thereby increasing the computational amount, and it is inferable that the predetermined length 11 to 14 is the intermediate-value. In addition, the length of the base sequence inputted by the input unit for base sequence is not limited to 19 or 20, and may be 15 to 60 for practical search. Note that even if the length is more than 61, the performance does not always degrade, and it was confirmed that as the length of the inputted base sequence becomes larger, the performance gradually degrades. Accordingly, it was confirmed that the present invention is useful for determination of sequence of oligo-DNA, of which length is about 60.

Hereinabove, although the search for the gene base sequence stored in the database has been described, the applicability of the present invention is not limited to the gene base sequence, and the present invention is applicable to general search for character strings etc. Therefore, the gene base sequence is a sequence, in which four bases are arranged one-dimensionally, so that by regarding the respective bases as an alphabet composing a character string, it becomes possible to regard the gene base sequence as the character string. In addition, as described above, the point that the number of bases is four should not be the limitation for the applicability of the present invention to the general character string.

Therefore, according to the present invention, it becomes possible to search for a character string similar to the inputted character string. Here, the term 'similar' corresponds to a character string having a predetermined hamming distance from the inputted character string, or to a character string having a hamming distance, which is less than the predetermined hamming distance, from the inputted character string.

Therefore, the following apparatus for searching for character string is provided. Hence, the apparatus for searching for character string, which searches for a similar character string, which has same length as, and similar to a character string to be inputted by using an index, which is for searching a database storing a character string, in which alphabets are arranged one-dimensionally, and is for searching for a position in said character string stored in said database, at which a character string having a predetermined length appears, comprising the input unit for character string, which inputs a character string having length longer than said predetermined length; the input unit for hamming distance, which inputs hamming distance, which indicates number of alphabets to be substituted to mismatching alphabets; the specifying unit, which specifies two different partial character strings, which are partial character strings of said inputted character string and have said predetermined length, and the other portion; the assignment unit, which distributes and assigns the hamming distance inputted by said input unit for hamming distance to the partial character string and to the other portion specified by said specifying unit; the selection unit, which selects the partial character string having non-larger total number of substituted character strings generated by substitution for said partial character string, in which the alphabets of the number indicated by the hamming distance assigned by said assignment unit are substituted to the mismatching alphabets, from the two partial character strings specified by said specifying unit; the generation unit for substituted character string, which generates a substituted character string, which has the hamming distance assigned by said assignment unit, for the partial character string selected by said selection unit; and the search unit, which carries out search using said index and the substituted character string generated by said generation unit for substituted character string as a search key, can be provided.

In addition, if the alphabet in the character string is a peptide, the present invention is applicable to searching for a peptide similar to an inputted peptide sequence.

As the tenth embodiment of the present invention, the apparatus for searching for base sequence according to any one of the first to eighth embodiments, which is improved for searching for a repeated sequence, will be described.

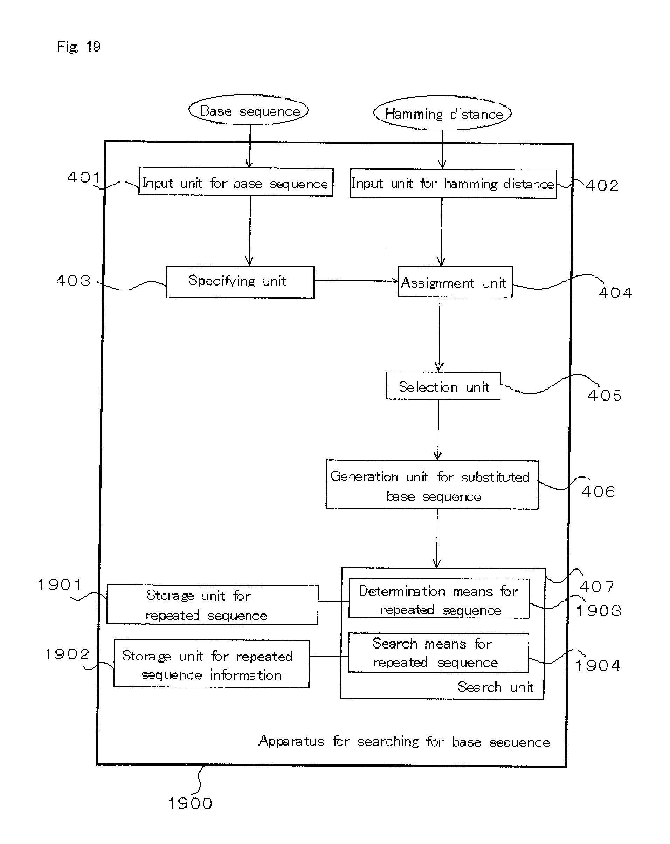

Fig. 19 is a functional block diagram of the apparatus for searching for base sequence of the tenth embodiment of the present invention. The apparatus for searching for base sequence of the tenth embodiment has a configuration, in which the apparatus for searching for base sequence according to any one of the first to eighth embodiments comprises the 'storage unit for repeated sequence' 1901, the 'storage unit for repeated sequence information' 1902, the 'determination means for repeated sequence' 1903, and the 'search means for repeated sequence' 1904. Fig. 19 is a functional block diagram of the apparatus for searching for base sequence according to the first embodiment comprising these units and means.

The 'storage unit for repeated sequence' 1901 stores base sequence of said predetermined length appearing repeatedly in the gene base sequence. The 'said predetermined length' is a value determined by the index used by the apparatus for searching for base sequence, and is the length of base sequence searchable by the index, in which the position of a gene base sequence is searched for in the base sequence.

It is known that the same base sequence repeatedly appears in a gene base sequence, and depending on the base sequence, the base sequence, of which the number of types is small, though, repeatedly appears in the gene base sequence a large amount of times. If the substituted base sequence generated by the generation unit for substituted base sequence 406 appears in the gene base sequence such a large amount of times, the processing efficiency in the apparatus for searching for base sequence according to any one of the first to eighth embodiments is lowered. Accordingly, in the tenth embodiment, the case, in which the substituted base sequence generated by the generation unit for substituted base sequence 406 repeatedly appears in the gene base sequence, is specifically addressed. For this reason, at the outset, the storage unit for repeated sequence 1901 stores the base sequence appearing repeatedly in the gene base sequence.

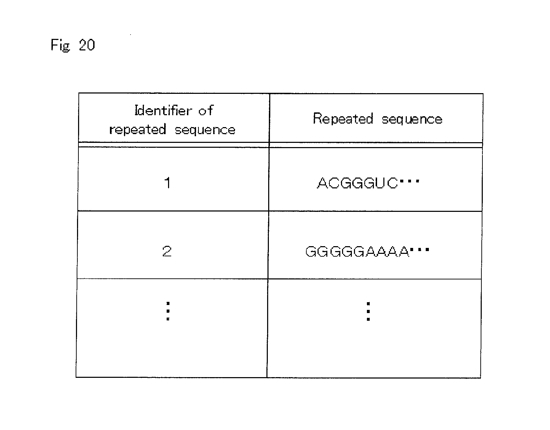

Fig. 20 is a diagram exemplifying that the repeated sequence is stored in the table. The identifier for uniquely identifying the base sequence appearing repeatedly in the gene base sequence and the base sequence are stored in the same row, so that the identifier and the base sequence are correlated and stored.

The 'storage unit for repeated sequence information' 1902 stores repeated sequence information. The repeated sequence information is information, in which the base sequence stored by said storage unit for repeated sequence is correlated with an position of appearance of the base sequence in said gene base sequence.

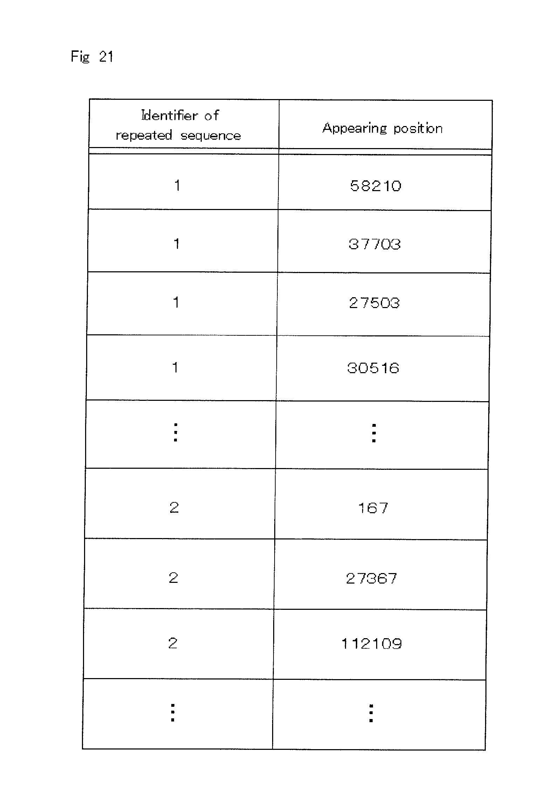

Fig. 21 is a diagram exemplifying the table storing the repeated sequence information. In this table, the identifier used in the table of Fig. 20 and the position of appearance of the base sequence in the gene base sequence are stored in the same row, thereby correlating them. In the column named as 'Identifier of repeated base sequence', the identifier is stored, and in the column named as 'Position of appearance', the position of appearance of the base sequence in the gene base sequence is stored.

The 'determination means for repeated sequence' 1903 determines whether the substituted base sequence is stored by the storage unit for repeated sequence 1901. For example, it is examined whether the substituted base sequence is stored in the column named 'Repeated sequence' in the table of Fig. 20. This processing can be carried out at a high speed by using the index (e.g., consisted of B⁺ tree) as 'value', which has the identifier stored in the column named 'Identifier of repeated sequence'. Note that the base sequence, which is determined by the determination means for repeated sequence 1903 that it is stored in the storage unit for repeated sequence 1901, is called the repeated sequence.

The 'search means for repeated sequence' 1904 carries out search based on the repeated sequence information stored in the storage unit for repeated sequence information, if it is determined by the determination means for repeated sequence that the substituted base sequence is stored by the storage unit for repeated sequence. For example, from the table of Fig. 20, the identifier stored in the column named as Identifier of repeated sequence is acquired, from Fig. 21, the position of appearance is acquired, the previous and subsequent base sequences are acquired, so that it is determined whether the hamming distance between the base sequence and the inputted base sequence is less than or equal to the predetermined hamming distance, thereby carrying out search.

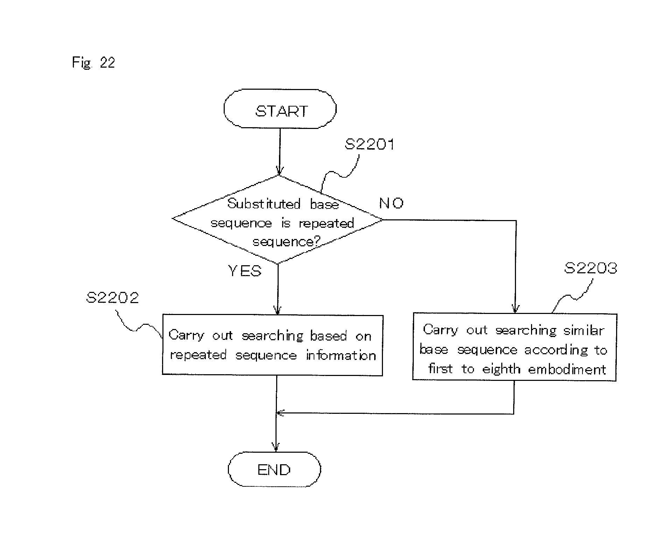

Fig. 22 is a flow chart of processes by the search unit of the apparatus for searching for base sequence of Fig. 19 of the tenth embodiment of the present invention. In the step S2201, it is determined by the determination means for repeated sequence whether the substituted base sequence is the repeated sequence. If it is the repeated sequence (i.e., the case of YES in the step S2201), the processing branches to the step S2202, and by the search means for repeated sequence1904, the search is carried out based on the repeated sequence information. If not, (i.e., the case of NO in the step S2201), the processing branches to the step S2203, and the search for similar base sequence is carried out according to the first to eighth embodiments. In addition, the search may be carried out only in the case where it is not the repeated base sequence, not in the case where it is the repeated base sequence.

According to the tenth embodiment, if the substituted base sequence is the repeated sequence, the search processing for repeated sequence is carried out, thereby preventing degradation of search speed caused by the repeated sequence.

As the eleventh embodiment of the present invention, the apparatus for searching for base sequence, which stores the search result of similar base sequence, will be described.

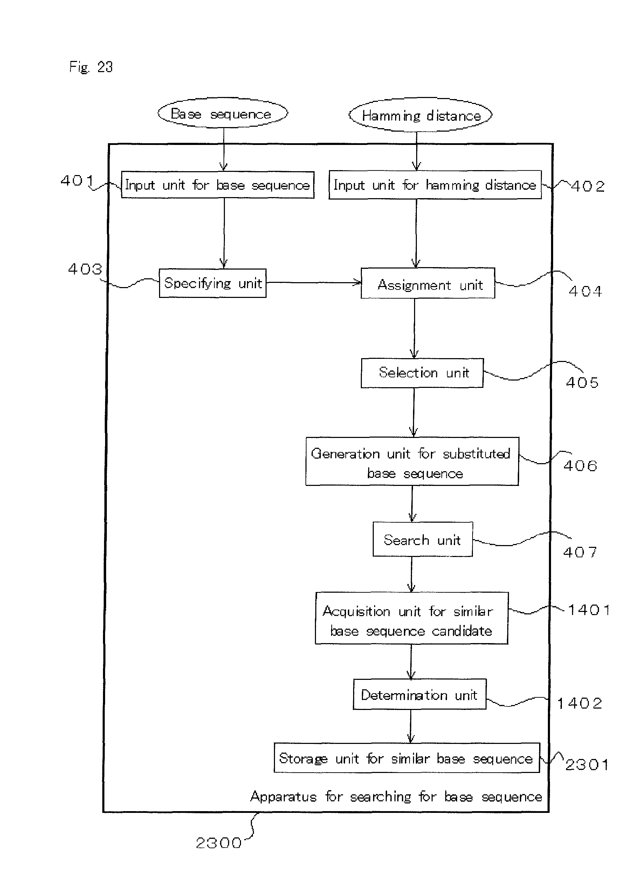

Fig. 23 is a functional block diagram of the apparatus for searching for base sequence of the eleventh embodiment of the present invention. The apparatus for searching for base sequence of the eleventh embodiment has a configuration, in which the apparatus for searching for base sequence according to any one of the fourth to seventh embodiments comprises the storage unit for similar base sequence 2301. Fig. 23 is a functional block diagram showing the case where the apparatus for searching for base sequence of the fourth embodiment comprises the storage unit for similar base sequence 2301.

The 'storage unit for similar base sequence' 2301 correlates (1) the inputted base sequence, (2) the hamming distance between the inputted base sequence and the similar base sequence candidate, with (3) the similar base sequence candidate, and stores them, if it is determined by the determination unit 1402 that the hamming distance between the inputted base sequence and the similar base sequence candidate acquired by said acquisition unit for similar base sequence candidate 1401 is less than or equal to the hamming distance inputted by the input unit for hamming distance 402.

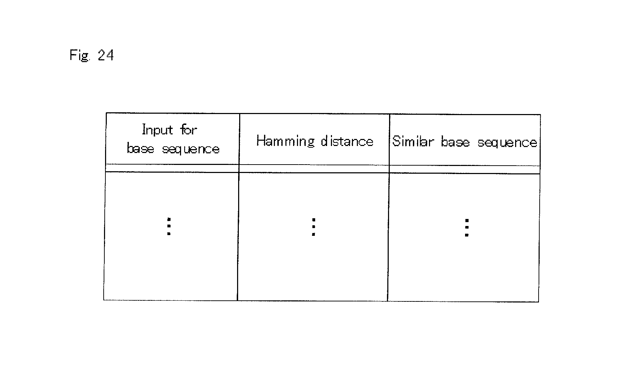

Fig. 24 is a diagram showing the structure of the table, in which (1) the inputted base sequence, (2) the hamming distance between the inputted base sequence and the similar base sequence candidate, and (3) the similar base sequence are correlated and stored. To the columns named 'Inputted base sequence', 'Hamming distance', and 'Similar base sequence', (1) the inputted base sequence, (2) the hamming distance between the inputted base sequence and the similar base sequence candidate, and (3) the similar base sequence are stored, respectively.

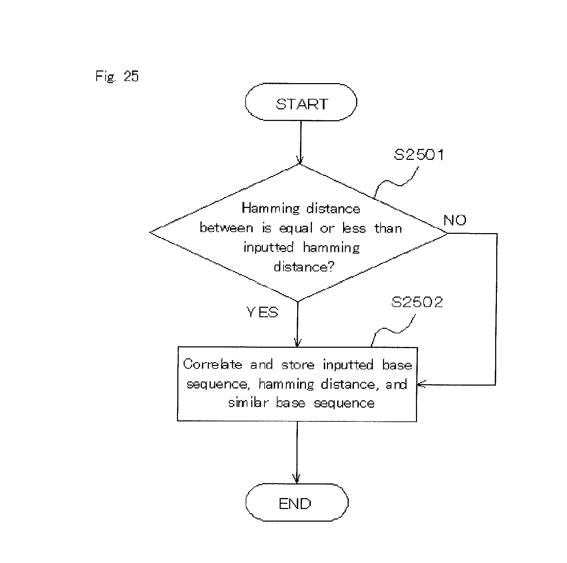

Fig. 25 is a flow chart of processes by the determination unit and the storage unit for similar base sequence of the apparatus for searching for base sequence of the eleventh embodiment. In the step S2501, it is determined by the determination unit whether the hamming distance between the inputted base sequence and the similar base sequence candidate is the inputted hamming distance. If so, the processing branches to YES in the step S2501, and in the step S2502, (1) the inputted base sequence, (2) the hamming distance, and (3) the similar base sequence are correlated and stored by the storage unit for similar base sequence 2301. If the processing branches to NO in the step S2501, the step S2502 is not carried out.

According to the eleventh embodiment, since the search result of the apparatus for searching for base sequence is stored in the storage unit for similar base sequence 2301, by searching the information stored in the storage unit for similar base sequence 2301, it is determined whether the search on the inputted base sequence and for the hamming distance, which are the same as the subjects to be searched for, has already been carried out, so that it becomes possible to search for the similar base sequence efficiently.

The apparatus for searching for base sequence of the eleventh embodiment is beneficial, for example, to the case where the search service is provided to many users via the internet etc. For example, in cases where the first person carried out search, and after that, the second person carried out the same search, the result of the search by the first person is used for the search by the second person, so that it becomes possible to reduce the response time and the burden of the apparatus for searching for base sequence.

As the twelfth embodiment of the present invention, the apparatus for searching for base sequence, which computes the association rate, will be described. Here, the term 'association rate' is a value indicating that two types of base sequences are concatenated at what percentage, in cases where two types of base sequences are placed in the fluid circumstance such as liquid. Such value can be computed by carrying out a physicochemical computation using base sequence. For example, the computational method is disclosed in the aforementioned Non-patent document 1.

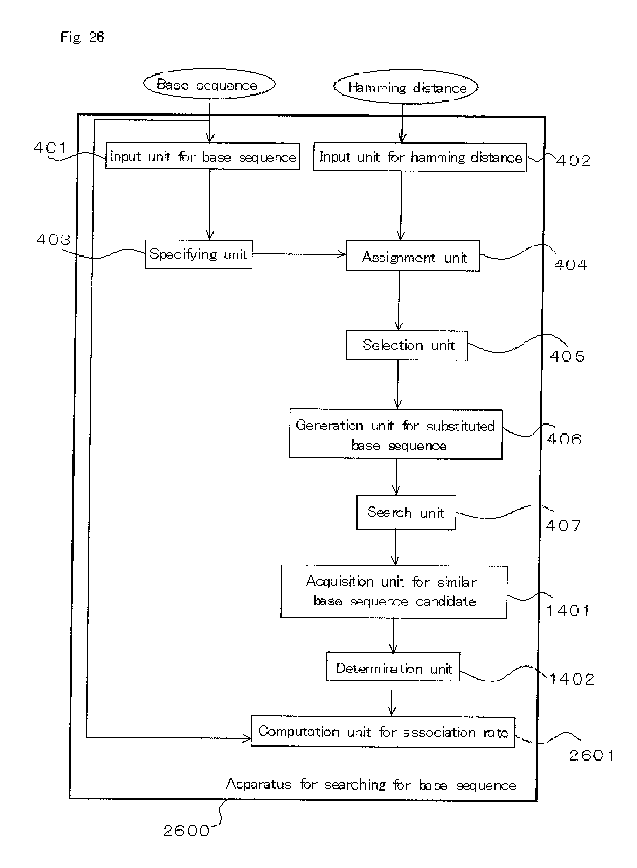

Fig. 26 is a functional block diagram of the apparatus for searching for base sequence of the twelfth embodiment of the present invention. The apparatus for searching for base sequence of the twelfth embodiment has the configuration, in which the apparatus for searching for base sequence according to any one of the fourth to seventh embodiments comprises the computation unit for association rate 2601. Fig. 26 is a functional block diagram showing the case where the apparatus for searching for base sequence of the fourth embodiment comprises the computation unit for association rate 2601.

The 'computation unit for association rate' 2601 computes association rate between (1) the inputted base sequence inputted by the input unit for base sequence 401 and (2) the similar base sequence candidate acquired by the acquisition unit for similar base sequence candidate 1401, if it is determined that the hamming distance between the similar base sequence candidate acquired by the acquisition unit for similar base sequence candidate 1401 and the inputted base sequence inputted by the input unit for base sequence 401 is less than or equal to the hamming distance inputted by the input unit for hamming distance 402. For example, conditions such as the temperature of a liquid and pH are preliminarily set, and the physicochemical computation of the association rate under the condition is carried out. Note that when the association rate is computed, the base forming the inputted base sequence, or the base forming the substituted base sequence is substituted to the complementary base.

According to the apparatus for searching for base sequence of the twelfth embodiment of the present invention, it becomes possible to efficiently search for the base sequence having the hamming distance between the base sequence and the inputted base sequence, which is less than or equal to the predetermined hamming distance, and to further acquire the association rate in cases where the wet experiment is actually carried out, thereby enabling estimation of the experimental result or an effect of medicine using RNA interference.

As the thirteenth embodiment of the present invention, the apparatus for searching for base sequence, which searches for the base sequence usable for control in the wet experiment etc., will be described.

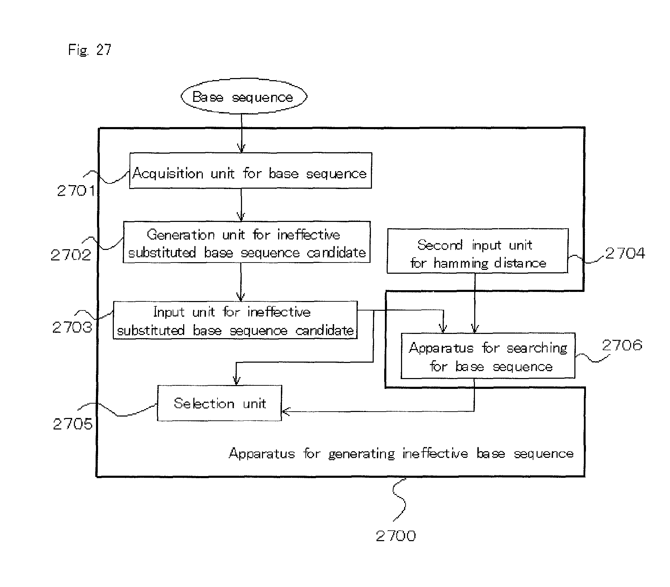

Fig. 27 is a functional block diagram of the apparatus for generating ineffective base sequence of the thirteenth embodiment of the present invention. The apparatus for generating ineffective base sequence 2700 comprises the acquisition unit for base sequence 2701, the generation unit for ineffective substituted base sequence candidate 2702, the input unit for ineffective substituted base sequence candidate 2703, the second input unit for hamming distance 2704, and the selection unit 2705.

The 'acquisition unit for base sequence' 2701 acquires a base sequence having a length longer than said predetermined length. As described in the tenth embodiment, the term 'said predetermined length' is a value determined by the index used by the apparatus for searching for base sequence according to any one of the fourth to seventh embodiments, and is the length of base sequence searchable by the index, in which the position of a gene base sequence is searched for in the base sequence.

The acquisition unit for base sequence is, for example, connected to the client apparatus via the communication network, and acquires the base sequence inputted to the WEB browser etc. operating in the client apparatus. The base sequence acquired by the acquisition unit for base sequence 2701 is, for example, the base sequence, which is proved to suppress the function of the target mRNA.

The 'generation unit for ineffective substituted base sequence candidate' 2702 generates ineffective substituted base sequence candidate. The term 'ineffective substituted base sequence candidate' is a base sequence acquired by substituting a predetermined number of bases among the bases of the base sequence acquired by the acquisition unit for base sequence. For example, if the length of the base sequence is 21 and the predetermined number of bases is 3, (4-1)³₂₁C₃ ineffective substituted base sequence candidates are generated ('4' of '(4-1)' indicates that the number of base types is 4). In addition, the base sequence, which is estimated to have a low association rate with the base sequence of target mRNA, may be generated based on the specific knowledge, not all ineffective substituted base sequence candidates. In addition, the ineffective substituted base sequence candidate may be generated using the sequence, which appears few times.

The 'input unit for ineffective substituted base sequence candidate' 2703 inputs the ineffective substituted base sequence candidate generated by the generation unit for ineffective substituted base sequence candidate 2702 to the apparatus for searching for base sequence according to the twelfth embodiment. For example, if the apparatus for generating ineffective base sequence and the apparatus for searching for base sequence of the twelfth embodiment are connected via LAN etc., the information indicating the ineffective substituted base sequence candidate is transmitted to the apparatus for searching for base sequence of the twelfth embodiment.

The 'second input unit for hamming distance' 2704 inputs a predetermined hamming distance to the apparatus for searching for base sequence 2706, to which the input unit for ineffective substituted base sequence candidate 2703 has inputted the ineffective substituted base sequence candidate. For example, the predetermined hamming distance is inputted upon the input unit for ineffective substituted base sequence candidate 2703 inputs the ineffective substituted base sequence candidate.

The 'selection unit' 2705 selects the base sequence having a low association rate from the ineffective substituted base sequence candidates, in which the base sequence is acquired by the apparatus for searching for base sequence 2706 according to the input by said input unit for ineffective substituted base sequence candidate, and to the input by the second input unit for hamming distance 2704. For example, if the association rate between a certain ineffective substituted base sequence candidate and a similar base sequence, similar thereto is 50%, and the association rate between another ineffective substituted base sequence candidate and a similar base sequence, similar thereto is 10%, the latter ineffective substituted base sequence candidate is selected, and is displayed etc. as the ineffective base sequence for a user of the apparatus for generating ineffective substituted base sequence.

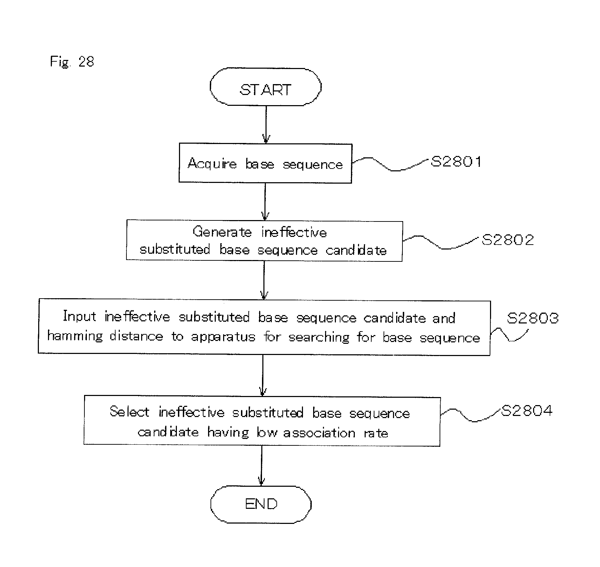

Fig. 28 is a flow chart of processes by the apparatus for generating ineffective base sequence of the thirteenth embodiment. In the step S2801, the base sequence is acquired by the acquisition unit for base sequence 2701. In the step S2802, the ineffective substituted base sequence candidate is generated by the generation unit for ineffective substituted base sequence candidate 2702. In the step S2803, the ineffective substituted base sequence candidate and the hamming distance are inputted to the apparatus for searching for base sequence 2706. The step S2803 is respectively carried out once for each ineffective substituted base sequence candidate, and the association rate for each ineffective substituted base sequence candidate is acquired. In the step S2804, the ineffective substituted base sequence candidate having a low association rate is selected by the selection unit 2705.

According to the thirteenth embodiment, it becomes possible to select the base sequence, which is similar to the provided base sequence, and has a low association rate. The selected base sequence is inferable to be the ineffective base sequence, so that it is usable for the control etc in the wet experiment.

As the fourteenth embodiment of the present invention, the alignment apparatus for base sequence using the apparatus for searching for base sequence of the present invention will be described.

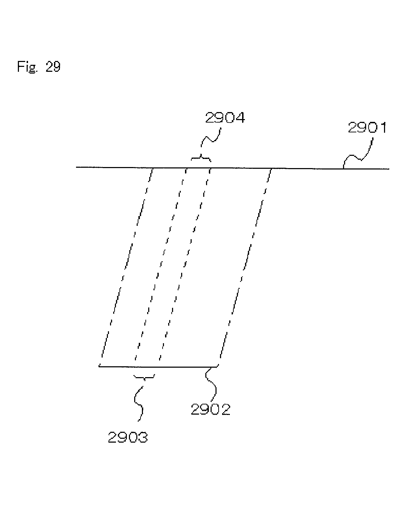

Fig. 29 is a schematic diagram of the processes by the apparatus of the fourteenth embodiment of the present invention. If the gene base sequence 2901 exists, it is desired to know that the base sequence similar to the base sequence 2902 exists at what position in the gene base sequence 2901. In this case, the partial sequence 2903 of the base sequence 2902 is acquired. The length of the partial sequence 2903 is suitable for the apparatus for searching for base sequence of the present invention, and is preferably 15 to 25 By using the apparatus for searching for base sequence of the present invention, the similar base sequence 2904, which is similar to the partial sequence 2903, is detected in the gene base sequence 2901. After that, by using the conventional method such as dynamic programming, the previous and subsequent base sequences of the partial sequence 2903, and of the similar base sequence 2904 are compared. By such operations, it becomes possible to efficiently know that the base sequence similar to the base sequence 2902 exists at what position in the gene base sequence 2901.

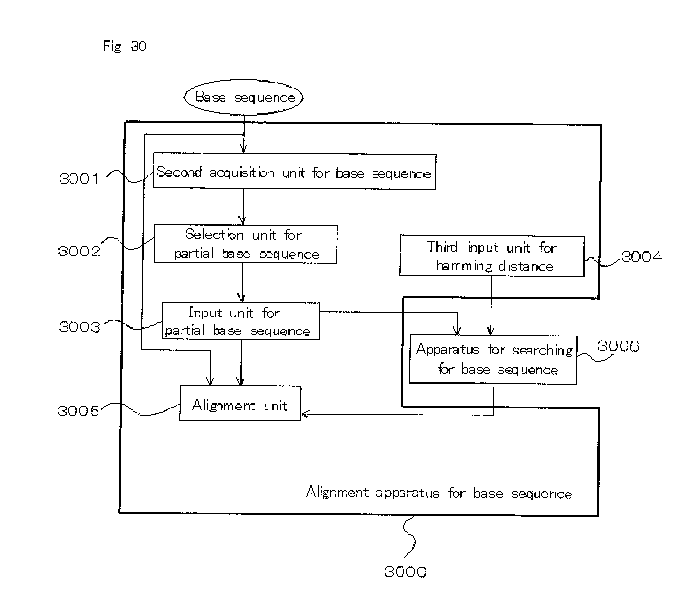

Fig. 30 is a functional block diagram of the alignment apparatus for base sequence of the fourteenth embodiment of the present invention. The alignment apparatus for base sequence comprises the second acquisition unit for base sequence 3001, the selection unit for partial base sequence 3002, the input unit for partial base sequence 3003, the third input unit for hamming distance 3004, and the alignment unit 3005.

The 'second acquisition unit for base sequence' 3001 acquires a base sequence having a length longer than said predetermined length.

The 'selection unit for partial base sequence' 3002 selects a partial base sequence, which is a portion of the base sequence acquired by said second acquisition unit for base sequence 3001. For example, the partial base sequence, of which the length is 15 to 25, is selected from the base sequence acquired by said second acquisition unit for base sequence 3001. The partial base sequence to be acquired is preferably not the repeated sequence as described in the twelfth embodiment. The reason for this is that many candidates for alignment are detected, so that the step S3104, which will be described hereinbelow, is required to be carried out many times. Accordingly, as in the twelfth embodiment, the storage for repeated sequence is comprised of the alignment apparatus for base sequence, and by referring the content stored by the storage for repeated sequence, the partial base sequence may be acquired.

The 'input unit for partial base sequence' 3003 inputs the partial base sequence selected by the selection unit for partial base sequence to the apparatus for searching for base sequence according to any one of the fourth to eighth embodiments 3006.

The 'third input unit for hamming distance' 3004 inputs a predetermined hamming distance to the apparatus for searching for base sequence 3006, to which the input unit for partial base sequence has inputted the partial base sequence. According to the respective inputs by the input unit for partial base sequence 3003 and by the third input unit for hamming distance 3004, the similar base sequence, which is similar to the partial base sequence, is determined, thereby determining the position in the gene base sequence.

The 'alignment unit' 3005 aligns the base sequence acquired by the second acquisition unit for base sequence 3001 to the gene base sequence based on the search result acquired by the apparatus for searching for base sequence 3006 according to both the input by the input unit for partial base sequence 3003, and the third input unit for hamming distance 3004. For example, if the partial base sequence is the portion indicated by number 2903, and it is proved by the apparatus for searching for base sequence 3006 that the similar base sequence, which is similar to the partial base sequence, is the portion indicated by number 2904, the score value etc., which indicates the similarity between the previous and subsequent bases of the base sequence indicated by number 2904 and the base sequence indicated by number 2902, is computed using the dynamic programming method etc.

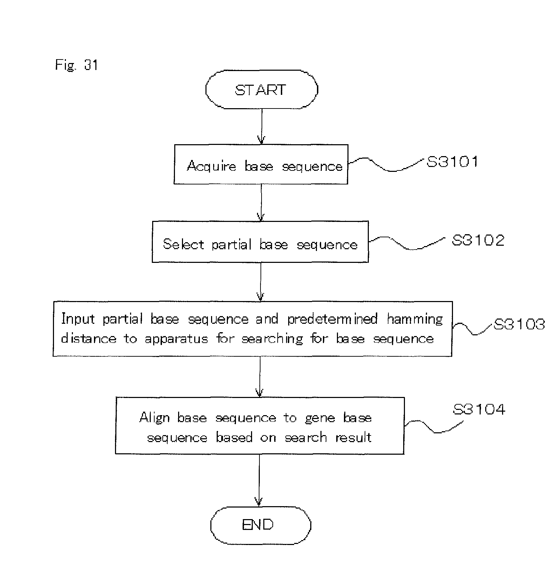

Fig. 31 is a flow chart of processes by the alignment apparatus for base sequence of the fourteenth embodiment of the present invention. In the step S3101, the second acquisition unit for base sequence 3001 acquires the base sequence. In the step S3102, the selection unit for partial base sequence 3002 selects a partial base sequence. In the step S3103, the input unit for partial base sequence 3003 and the third input unit for hamming distance 3004 inputs the partial base sequence, and the predetermined hamming distance to the apparatus for searching for base sequence 3006, respectively. In the step S3104, the base sequence is aligned to the gene base sequence based on the search result by the apparatus for searching for base sequence 3006. The step S3104 is repeatedly carried out according to the search result acquired by the step S3103.

In the conventional alignment method, BLAST has been used. However, in BLAST, for example, the base sequence having the same successive 7mer is searched, so that it is detected that the similar base sequence appears at what position in the gene base sequence. In some cases, this makes it difficult to carry out an accurate alignment. According to the present invention, a similar base sequence, which is similar to the partial base sequence, is searched for, so that it becomes possible to carry out more accurate alignment.

### Industrial Applicability

According to the apparatus for searching for base sequence and the method for searching for base sequence of the present invention, the computational amount required for the search is reduced, and the hamming distance is less than the predetermined value, so that there is no failure in detecting the existence of similar base sequences. This is beneficial in designing base sequence etc. For example, in cases where the apparatus for searching for base sequence and the method for searching for base sequence of the present invention are applied to the designing base sequence of ₛᵢRNA, specifically, in cases where they are applied in combination with the various predetermined guidelines, which implement designing _{Si}RNA having high RNA-interference (RNAi) effect (specifically, the guidelines by Ui-Tei et al., 'Guidelines for the selection of highly effective siRNA sequences for mammalian and chick RNA interference', Ui-Tei,K., Naito,Y., Takahashi,F., Haraguchi,T., Ohki-Hamazaki,H., Juni,A., Ueda,R. and Saigo,K, Nucleic Acids Research, 2004, Vol. 32, No.3, 936-948), they are beneficial with the objective of reducing required working time, with appropriate designing.

## Claims

1. An apparatus for searching for base sequence, which searches for a similar base sequence, which has same length, and is similar to a base sequence to be inputted, using an index, which is for searching a database storing a gene base sequence indicating gene information, and is for searching for a position in said gene base sequence, at which a base sequence having a predetermined length appears, comprising:
an input unit for base sequence, which inputs a base sequence having a length longer than said predetermined length;
an input unit for hamming distance, which inputs hamming distance, which indicates number of bases to be substituted to mismatching bases;
a specifying unit, which specifies two different partial sequences, which are partial sequences of said inputted base sequence and have said predetermined length, and the other portion;
an assignment unit, which distributes and assigns the hamming distance inputted by said input unit for hamming distance to the partial sequence and to the other portion specified by said specifying unit;
a selection unit, which selects the partial sequence having a non-larger total number of substituted base sequences generated by substitution for said partial sequence, in which the bases of the number indicated by the hamming distance assigned by said assignment unit are substituted to the mismatching bases, from the two partial sequences specified by said specifying unit;
a generation unit for substituted base sequence, which generates a substituted base sequence, which has the hamming distance assigned by said assignment unit, for the partial sequence selected by said selector; and
a search unit, which carries out a search using said index and the substituted base sequence generated by said generator for substituted base sequence as a search key.

2. The apparatus for searching for base sequence according to Claim 1, wherein said specifying unit comprising:
a first specifying means, in which, if number of bases of the base sequence inputted by said input unit for a base sequence is equal to or less than twice of said predetermined length, one end of one partial sequence of said two partial sequences is conformed to one end of said inputted base sequence, and one end of another partial sequence of said two partial sequences is conformed to another end of said inputted base sequence, so that the other portion does not exist and is not specified.

3. The apparatus for searching for base sequence according to Claim 1 or 2, wherein said specifying unit comprising:
a second specifying means, in which, if number of bases of the base sequence inputted by said input unit for base sequence is more than twice of said predetermined length,
said two partial sequences do not overlap with each other, and said two partial sequences are specified.

4. The apparatus for searching for base sequence according to any one of Claims 1 to 3, comprising:
an acquisition unit for similar base sequence candidate, which acquires a similar base sequence candidate, which is a base sequence including said substituted base sequence and appearing in a gene base sequence, based on the search result by said search unit; and
a determination unit, which determines whether the hamming distance between the similar base sequence candidate acquired by said acquisition unit for similar base sequence candidate and said inputted base sequence is equal to or less than the hamming distance inputted by said input unit for hamming distance.

5. The apparatus for searching for base sequence according to Claim 4, comprising:
an input unit for mismatching base pair, which specifies mismatching base pair, wherein
the search unit carries out searching and computing the hamming distance based on the base pair inputted by the input unit for mismatching base pair.

6. The apparatus for searching for base sequence according to Claim 4 or 5, comprising:
an input unit for distribution of matches, which inputs distribution information indicating distribution of matches between the corresponding bases in the base sequence inputted by said input unit for base sequence and the similar base sequence, wherein
said determination unit comprises,
a determination means for distribution, which determines whether the distribution information inputted by said input unit for distribution of matches has been fulfilled.

7. The apparatus for searching for base sequence according to Claim 6, wherein
the distribution information inputted by said input unit for distribution of matches is lower limit of length of successive matching bases between the base sequence and the similar base sequence.

8. The apparatus for searching for base sequence according to any one of Claims 1 to 7, wherein
the length of the base sequence inputted by said input unit for base sequence is 15 to 60, and said predetermined length is 11 to 14.

9. A method for searching for base sequence, which searches for a similar base sequence, which has same length, and is similar to base sequence to be inputted, by using an index, which is for searching a database storing a gene base sequence indicating gene information, and is for searching for a position in said gene base sequence, at which a base sequence having a predetermined length appears, comprising:
a step of inputting base sequence, which inputs a base sequence having a length longer than said predetermined length;
a step of inputting hamming distance, which inputs hamming distance, which indicates number of mismatching bases to be substituted;
a step of specifying, which specifies two different partial sequences, which are partial sequences of said inputted base sequence, and have said predetermined length, and the other portion;
a step of assigning, which distributes and assigns the hamming distance inputted by said input unit for hamming distance to the partial sequence and to the other portion specified by said specifying unit;
a step of selecting, which selects the partial sequence having a non-larger total number of substituted base sequences generated by substitution for said partial sequence, in which the bases of the number indicated by the hamming distance assigned by said assignment unit are substituted to the mismatching bases, from the two partial sequences specified by said specifying unit;
a step of generating substituted base sequence, which generates a substituted base sequence, which has the hamming distance assigned by said assignment unit, for the partial sequence selected by said selector; and
a step of searching, which carries out a search using said index and the substituted base sequence generated by said generator for substituted base sequence as a search key.

10. An apparatus for searching for character string, which searches for a similar character string, which has same length, and is similar to a character string to be inputted, by using an index, which is for searching a database storing a character string, in which alphabets are arranged one-dimensionally, and is for searching for a position in said character string stored in said database, at which a character string having a predetermined length appears, comprising:
an input unit for character string, which inputs a character string having a length longer than said predetermined length;
an input unit for hamming distance, which inputs hamming distance, which indicates number of alphabets to be substituted to mismatching alphabets;
a specifying unit, which specifies two different partial character strings, which are partial character strings of said inputted character string and have said predetermined length, and the other portion;
an assignment unit, which distributes and assigns the hamming distance inputted by said input unit for hamming distance to the partial character string and to the other portion specified by said specifying unit;
a selection unit, which selects the partial character string having a non-larger total number of substituted character strings generated by substitution for said partial character string, in which the alphabets of the number indicated by the hamming distance assigned by said assignment unit are substituted to the mismatching alphabets, from the two partial character strings specified by said specifying unit;
a generation unit for substituted character string, which generates a substituted character string, which has the hamming distance assigned by said assignment unit, for the partial character string selected by said selection unit; and
a search unit, which carries out a search using said index and the substituted character string generated by said generation unit for substituted character string as a search key.

11. The apparatus for searching for character string according to Claim 10, wherein
said character string is a peptide sequence.

12. The apparatus for searching for base sequence according to any one of Claims I to 8, comprising:
a storage unit for repeated sequence, which stores base sequence of said predetermined length appearing repeatedly in the gene base sequence;
a storage unit for repeated sequence information, which stores repeated sequence information, in which the base sequence stored by said storage unit for repeated sequence is correlated with a position in said gene base sequence, at which the base sequence appears, wherein
said search unit comprises,
a determination means for repeated sequence, which determines whether said substituted base sequence is stored by said storage unit for repeated sequence, and
a search means for repeated sequence, which carries out search based on the repeated sequence information stored in said storage unit for repeated sequence information, if it is determined by said determination means for repeated sequence that said substituted base sequence is stored by said storage unit for repeated sequence.

13. The apparatus for searching for base sequence according to any one of Claims 4 to 7, comprising:
a storage unit for similar base sequence, which correlates said inputted base sequence, the hamming distance between said inputted base sequence and a similar base sequence candidate, with the similar base sequence candidate, and stores them, if it is determined by said determination unit that the hamming distance between said inputted base sequence and the similar base sequence candidate acquired by said acquisition unit for similar base sequence candidate is less than or equal to the hamming distance inputted by said input unit for hamming distance.

14. The apparatus for searching for base sequence according to any one of Claims 4 to 7, comprising:
a computation unit for association rate, which computes association rate between said base sequence inputted by said input unit for base sequence and the similar base sequence candidate acquired by said acquisition unit for similar base sequence candidate, if it is determined by said determination unit that the hamming distance between the similar base sequence candidate acquired by said acquisition unit for similar base sequence candidate and said inputted base sequence is less than or equal to the hamming distance inputted by said input unit for hamming distance.

15. An apparatus for generating ineffective base sequence, comprising:
an acquisition unit for base sequence, which acquires a base sequence having a length longer than said predetermined length;
a generation unit for ineffective substituted base sequence candidate, which generates ineffective substituted base sequence candidate, which is a base sequence acquired by substituting a predetermined number of bases among the bases of the base sequence acquired by said acquisition unit for base sequence;
an input unit for ineffective substituted base sequence candidate, which inputs the ineffective substituted base sequence candidate generated by said generation unit for ineffective substituted base sequence candidate to the apparatus for searching for base sequence according to Claim 14;
a second input unit for hamming distance, which inputs a predetermined hamming distance to the apparatus for searching for base sequence, to which said input unit for ineffective substituted base sequence candidate has inputted the ineffective substituted base sequence candidate; and
a selection unit, which selects the base sequence having a low association rate from the ineffective substituted base sequence candidates generated by said generation unit for ineffective substituted base sequence candidate, in which the base sequence is acquired by said apparatus for searching for base sequence according to the input by said input unit for ineffective substituted base sequence candidate, and to the input by said second input unit for hamming distance.

16. An alignment apparatus for base sequence, comprising:
a second acquisition unit for base sequence, which acquires a base sequence having a length longer than said predetermined length;
a selection unit for partial base sequence, which selects a partial base sequence, which is a portion of the base sequence acquired by said second acquisition unit for base sequence;
an input unit for partial base sequence, which inputs the partial base sequence selected by said selection unit for partial base sequence to the apparatus for searching for base sequence according to any one of Claims 4 to 8;
a third input unit for hamming distance, which inputs a predetermined hamming distance to the apparatus for searching for base sequence, to which said input unit for partial base sequence has inputted the partial base sequence; and
an alignment unit, which aligns the base sequence acquired by said second acquisition unit for a base sequence to said gene base sequence based on the search result acquired by said apparatus for searching for base sequence according to the input by said input unit for partial base sequence, and to the input by said third input unit for hamming distance.
